# EUROPEAN PATENT SPECIFICATION

(11) **EP 1 315 727 B1**
(45) Date of publication and mention of the grant of the patent: **29.06.2005**
(21) Application number: 01980258.6
(22) Date of filing: 22.08.2001
(51) Int. Cl.: C07D 471/04, A61K 31/519, A61P 25/00, C07D 239/46

(54) **7-OXO PYRIDOPYRIMIDINES**
7-OXOPYRIDORYRIMIDINE
7-OXO PYRIDOPYRIMIDINES

(30) Priority: 31.08.2000 US 229577 P
(43) Date of publication of application: 04.06.2003
(73) Proprietor: F. Hoffmann-La Roche AG, 4070 Basel (CH)
(72) Inventor: ARZENO, Humberto, Bartolome, Cupertino, CA 95014 (US); CHEN, Jian, Jeffrey, Santa Clara, CA 95051 (US); DUNN, James, Patrick, Los Altos, CA 94022 (US); GOLDSTEIN, David, Michael, San Jose, CA 95138 (US); LIM, Julie, Anne, San Mateo, CA 94402 (US)
(74) Representative: Braun, Axel, Dr.
(86) International application number: PCT/EP2001/009688
(87) International publication number: WO 2002/018379

(56) References cited:
- WO-A-00/12497
- WO-A-02/18380
- WO-A-96/34867
- WO-A-97/44321
- US-A- 5 945 422

## Description

The present invention relates to 7-oxo-pyridopyrimidines. In particular, the present invention provides 2,6-disubstituted 7-oxo-pyrido[2,3-d]pyrimidines, a process for their manufacture, pharmaceutical preparations comprising the same, and methods for using the same.

Mitogen-activated protein kinases (MAP) is a family of proline-directed serine/threonine kinases that activate their substrates by dual phosphorylation. The kinases are activated by a variety of signals including nutritional and osmotic stress, UV light, growth factors, endotoxin and inflammatory cytokines. One group of MAP kinases is the p38 kinase group which includes various isoforms (*e*.*g*., p38α, p39β, p38γ and p38δ). The p38 kinases are responsible for phosphorylating and activating transcription factors as well as other kinases, and are activated by physical and chemical stress, pro-inflammatory cytokines and bacterial lipopolysaccharide.

More importantly, the products of the p38 phosphorylation have been shown to mediate the production of inflammatory cytokines, including TNF and IL-1, and cyclooxygenase-2. Each of these cytokines has been implicated in numerous disease states and conditions. For example, TNF-α is a cytokine produced primarily by activated monocytes and macrophages. Its excessive or unregulated production has been implicated as playing a causative role in the pathogenesis of rheumatoid arthritis. More recently, inhibition of TNF production has been shown to have broad application in the treatment of inflammation, inflammatory bowel disease, multiple sclerosis and asthma.

TNF has also been implicated in viral infections, such as HIV, influenza virus, and herpes virus including herpes simplex virus type-1 (HSV-1), herpes simplex virus type-2 (HSV-2), cytomegalovirus (CMV), varicella-zoster virus (VZV), Epstein-Barr virus, human herpes virus-6 (HHV-6), human herpesvirus-7 (HHV-7), human herpesvirus-8 (HHV-8), pseudorabies and rhinotracheitis, among others.

Similarly, IL-1 is produced by activated monocytes and macrophages, and plays a role in many pathophysiological responses including rheumatoid arthritis, fever and reduction of bone resorption.

Additionally, the involvement of p38 has been implicated in stroke, Alzheimer's disease, osteoarthritis, lung injury, septic shock, angiogenesis, dermatitis, psoriasis and atopic dermatitis, see e.g. J. Exp. Opin. Ther. Patents, (2000) 10(1).

Certain pyrido[2,3-d]pyrimidines are disclosed as inhibitors of protein tyrosine kinase mediated cellular proliferation in WO 96/34867.

The inhibition of these cytokines by inhibition of the p38 kinase is of benefit in controlling, reducing and alleviating many of these disease states.

One aspect of the present invention provides compounds represented by the Formula: 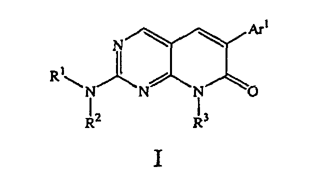 a prodrug or a pharmaceutically acceptable salt thereof,
in which:
R¹ is hydrogen or alkyl;
R² is -CR'R"-R^{a} (where R' and R" are hydrogen, hydroxyalkyl or alkyl with at least one being alkyl or hydroxyalkyl and R^{a} is hydroxyalkyl), R^{x}-S(O)₂-R^{y} (where R^{x} is alkyl and R^{y} is alkenyl), alkoxy-substituted alkyl, heterocyclylalkyl or C₄-C₅ cycloalkyl, wherein each of the hydroxy group present in R² can be independently in the form of an ester, a carbamate, a carbonate, or a sulfonate derivative; or

R¹ and R² together with the nitrogen atom to which they are attached form heterocyclyl;
R³ is hydrogen, alkyl, cycloalkyl, aryl, aralkyl, haloalkyl, heteroalkyl, cyanoalkyl, amino, monoalkylamino, dialkylamino, alkylene-C(O)-R (where R is hydrogen, alkyl, hydroxy, alkoxy, amino, monoalkylamino or dialkylamino) or acyl; and
Ar¹ is aryl.

The compounds of Formula I and their aforementioned salts are inhibitors of protein kinases, and exhibit effective activity against p38 *in vivo*. Therefore, the compounds can be used for the treatment of diseases mediated by the pro-inflammatory cytokines such as TNF and IL-1.

Thus, another aspect of the present invention provides methods for the treatment of p38 mediated diseases or conditions in which a therapeutically effective amount of a compound of Formula I is administered to a patient in need of such treatment.

Yet another aspect of the present invention provides methods for preparing the compounds described above.

Still yet another aspect of the present invention provides methods for preparing medicaments useful for the treatment of the p38 mediated diseases and conditions.

Unless otherwise stated, the following terms used in the specification and claims have the meanings given below:
"Acyl" means a radical -C(O)R, where R is hydrogen, alkyl, cycloalkyl, cycloalkylalkyl, phenyl or phenylalkyl wherein alkyl, cycloalkyl, cycloalkylalkyl, and phenylalkyl are as defined herein. Representative examples include, but are not limited to formyl, acetyl, cylcohexylcarbonyl, cyclohexylmethylcarbonyl, benzoyl, benzylcarbonyl, and the like.
"Acylamino" means a radical -NR'C(O)R, where R' is hydrogen or alkyl, and R is hydrogen, alkyl, cycloalkyl, cycloalkylalkyl, phenyl or phenylalkyl wherein alkyl, cycloalkyl, cycloalkylalkyl, and phenylalkyl are as defined herein. Representative examples include, but are not limited to formylamino, acetylamino, cylcohexylcarbonylamino, cyclohexylmethyl-carbonylamino, benzoylamino, benzylcarbonylamino, and the like.
"Alkoxy" means a radical -OR where R is an alkyl as defined herein e.g., methoxy, ethoxy, propoxy, butoxy and the like.
"Alkyl" means a linear saturated monovalent hydrocarbon radical of one to six carbon atoms or a branched saturated monovalent hydrocarbon radical of three to six carbon atoms, e.g., methyl, ethyl, propyl, 2-propyl, *n*-butyl, *iso*-butyl, *tert*-butyl, pentyl, and the like.
"Alkylene" means a linear saturated divalent hydrocarbon radical of one to six carbon atoms or a branched saturated divalent hydrocarbon radical of three to six carbon atoms, e.g., methylene, ethylene, 2,2-dimethylethylene, propylene, 2-methylpropylene, butylene, pentylene, and the like.
"Alkylthio" means a radical -SR where R is an alkyl as defined above e.g., methylthio, ethylthio, propylthio, butylthio, and the like.
"Aryl" means a monovalent monocyclic or bicyclic aromatic hydrocarbon radical which is optionally substituted independently with one or more substituents, preferably one, two or three, substituents preferably selected from the group consisting of alkyl, hydroxy, alkoxy, haloalkyl, haloalkoxy, heteroalkyl, halo, nitro, cyano, amino, monoalkylamino, dialkylamino, methylenedioxy, ethylenedioxy and acyl. More specifically the term aryl includes, but is not limited to, phenyl, chlorophenyl, methoxyphenyl, 1-naphthyl, 2-naphthyl, and the derivatives thereof, or fluorophenyl, or even mor specifically chlorophenyl or fluorophenyl.
"Cycloalkyl" refers to a saturated monovalent cyclic hydrocarbon radical of three to seven, preferably 3 to 5, ring carbons e.g., cyclopropyl, cyclobutyl, cyclohexyl, 4-methylcyclohexyl, and the like, preferably cyclopropyl, cyclobutyl or cyclohexyl.
"Dialkylamino" means a radical -NRR' where R and R' independently represent an alkyl, hydroxyalkyl, cycloalkyl, or cycloalkylalkyl group as defined herein. Representative examples include, but are not limited to dimethylamino, methylethylamino, di(1-methylethyl)amino, (cyclohexyl)(methyl)amino, (cyclohexyl)(ethyl)amino, (cyclohexyl)(propyl)amino, (cyclohexylmethyl)(methyl)amino, (cyclohexylmethyl)(ethyl)amino, and the like.

The term "each of the hydroxy group present in R² can be independently in the form of an ester, a carbamate, a carbonate or a sulfonate derivative" means that hydroxy group(s) (-OH) which are present in the R² group can be independently derivatized as R^{a}-C(=O)-O-, R^{a}R^{b}N-C(=O)-O-, R^{a}-O-C(=O)-O- or R^{a}-SO₂-O-, respectively, where R^{a} and R^{b} is independenlty hydrogen, alkyl, aryl or aralkyl as defined herein.

"Halo" means fluoro, chloro, bromo, or iodo, preferably fluoro and chloro.

"Haloalkyl" means alkyl substituted with one or more same or different halo atoms, e.g., -CH₂Cl, -CF₃, -CH₂CF₃, -CH₂CCl₃, and the like.

"Heteroalkyl" means an alkyl radical as defined herein wherein one, two or three hydrogen atoms have been replaced with a substituent independently selected from the group consisting of -OR^{a}, -NR^{b}R^{c}, and -S(O)ₙR^{d} (where n is an integer from 0 to 2), with the understanding that the point of attachment of the heteroalkyl radical is through a carbon atom, wherein R^{a} is hydrogen, acyl, alkyl, cycloalkyl, or cycloalkylalkyl; R^{b} and R^{c} are independently of each other hydrogen, acyl, alkyl, cycloalkyl, or cycloalkylalkyl, or R^{b} and R^{c} together forms cycloalkyl or arylcycloalkyl; and when n is 0, R^{d} is hydrogen, alkyl, cycloalkyl, or cycloalkylalkyl, and when n is 1 or 2, R^{d} is alkyl, cycloalkyl, cycloalkylalkyl, amino, acylamino, monoalkylamino, or dialkylamino. Representative examples include, but are not limited to, 2-hydroxyethyl, 3-hydroxypropyl, 2-hydroxy-1-hydroxymethylethyl, 2,3-dihydroxypropyl, 1-hydroxymethylethyl, 3-hydroxybutyl, 2,3-dihydroxybutyl, 2-hydroxy-1-methylpropyl, 2-aminoethyl, 3-aminopropyl, 2-methylsulfonylethyl, aminosulfonylmethyl, aminosulfonylethyl, aminosulfonylpropyl, methylaminosulfonylmethyl, methylaminosulfonylethyl, methylaminosulfonylpropyl, and the like.

"Heterocyclyl" means a saturated or unsaturated non-aromatic cyclic radical of 3 to 8 ring atoms in which one or two ring atoms are heteroatoms selected from N, O, or S(O)ₙ (where n is an integer from 0 to 2), the remaining ring atoms being C. More specifically "heterocyclyl" means a saturated non-aromatic cyclic radical of 5 to 6, preferably six ring atoms in which one or two ring atoms, preferably one ring atom is/are N- the remaining ring atoms are C-atoms. The heterocyclyl ring may be optionally substituted independently with one, two, or three substituents selected from alkyl, haloalkyl, heteroalkyl, halo, nitro, cyano, cyanoalkyl, hydroxy, alkoxy, amino, monoalkylamino, dialkylamino, aralkyl, -(X)ₙ-C(O)R (where, X is O or NR', n is 0 or 1, R is hydrogen, alkyl, haloalkyl, hydroxy (when n is 0), alkoxy, amino, monoalkylamino, dialkylamino or optionally substituted phenyl, and R' is H or alkyl), -alkylene-C(O)R (where R is OR or NR'R"and R is hydrogen, alkyl or haloalkyl, and R' and R" are independently hydrogen or alkyl), -alkylene-S(O)ₙ-R^{a} (where n is 0, 1 or 2, preferably 0, and R^{a} is alkyl) or -S(O)ₙR (where n is an integer from 0 to 2) such that when n is 0, R is hydrogen, alkyl, cycloalkyl, or cycloalkylalkyl, and when n is 1 or 2, R is alkyl, cycloalkyl, cycloalkylalkyl, amino, acylamino, monoalkylamino or dialkylamino. More specifically the term heterocyclyl includes, but is not limited to, tetrahydropyranyl, piperidino, N-methylpiperidin-3-yl, piperazino, N-methylpyrrolidin-3-yl, 3-pyrrolidino, morpholino, thiomorpholino, thiomorpholino-1-oxide, thiomorpholino-1,1-dioxide, pyrrolinyl, imidazolinyl, N-methanesulfonyl-piperidin-4-yl, and the derivatives thereof, preferably piperidinyl. Preferably "heterocyclyl" is substituted at the heteroatom with one substituent selected from alkyl, preferably substituted alkyl, wherein the substitution is amino, carbonyl, e.g. mono-or preferably dialkylaminocarbonyl,e.g. dimethylaminocarbonyl, carboxyl or alkyloxycarbonyl, e.g. methoxycarbonyl.

"Heterocyclylalkyl" means a radical -R^{a}R^{b} where R^{a} is an alkylene group and R^{b} is a heterocyclyl group as defined above with the understanding that R^{b} is attached to R^{a} via a carbon atom of the heterocyclyl ring, e.g., tetrahydropyran-2-ylmethyl, 2- or 3-piperidinylmethyl, and the like.

"Hydroxyalkyl" means an alkyl radical as defined herein, substituted with one or more, preferably one, two or three hydroxy groups more preferably one or two, provided that the same carbon atom does not carry more than one hydroxy group, preferably the substituents of R² as defined in examples 7-18 and in Table 1 compounds 7, 12, 13, 24 and 25. Representative examples include, but are not limited to, hydroxymethyl, 2-hydroxyethyl, 2-hydroxypropyl, 3-hydroxypropyl, 1-(hydroxymethyl)-2-methylpropyl, 2-hydroxybutyl, 3-hydroxybutyl, 4-hydroxybutyl, 2,3-dihydroxypropyl, 2-hydroxy-1-hydroxymethylethyl, 2,3-dihydroxybutyl, 3,4-dihydroxybutyl and 2-(hydroxymethyl)-3-hydroxypropyl, preferably 2-hydroxyethyl, 2,3-dihydroxypropyl and 1-(hydroxymethyl)-2-hydroxyethyl. Accordingly, as used herein, the term "hydroxyalkyl" is used to define a subset of heteroalkyl groups.

"Leaving group" has the meaning conventionally associated with it in synthetic organic chemistry, i.e., an atom or a group capable of being displaced by a nucleophile and includes halo (such as chloro, bromo, and iodo), alkanesulfonyloxy, arenesulfonyloxy, alkylcarbonyloxy (e.g., acetoxy), arylcarbonyloxy, mesyloxy, tosyloxy, trifluoromethanesulfonyloxy, aryloxy (e.g., 2,4-dinitrophenoxy), methoxy, N,O-dimethylhydroxylamino, and the like.

"Monoalkylamino" means a radical -NHR where R is an alkyl, hydroxyalkyl, cycloalkyl, or cycloalkylalkyl group as defined above, e.g., methylamino, (1-methylethyl)amino, hydroxymethylamino, cyclohexylamino, cyclohexylmethylamino, cyclohexylethylamino, and the like.

"Optionally substituted phenyl" means a phenyl ring which is optionally substituted independently with one or more substituents, preferably one or two substituents selected from the group consisting of alkyl, hydroxy, alkoxy, haloalkyl, haloalkoxy, heteroalkyl, halo, nitro, cyano, amino, methylenedioxy, ethylenedioxy, and acyl, preferably a phenyl ring which is substituated with one substituent which is preferably halogene, e.g. fluoro or chloro.

"Pharmaceutically acceptable excipient" means an excipient that is useful in preparing a pharmaceutical composition that is generally safe, non-toxic and neither biologically nor otherwise undesirable, and includes an excipient that is acceptable for veterinary use as well as human pharmaceutical use. A "pharmaceutically acceptable excipient" as used in the specification and claims includes both one and more than one such excipient.

"Pharmaceutically acceptable salt" of a compound means a salt that is pharmaceutically acceptable and that possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, tromethamine, N-methylglucamine, and the like.

The terms "pro-drug" and "prodrug" are used interchangeably herein and refer to any compound which releases an active parent drug according to Formula I in vivo when such prodrug is administered to a mammalian subject. Prodrugs of a compound of Formula I are prepared by modifying one or more functional group(s) present in the compound of Formula I in such a way that the modification(s) may be cleaved *in vivo* to release the parent compound. Prodrugs include compounds of Formula I wherein a hydroxy, amino, sulfhydryl, carboxy or carbonyl group in a compound of Formula I is bonded to any group that may be cleaved *in vivo* to regenerate the free hydroxyl, amino, or sulfhydryl group, respectively. Examples of prodrugs include, but are not limited to, esters (e.g., acetate, dialkylaminoacetates, formates, phosphates, sulfates, and benzoate derivatives), sulfonates and carbamates (e.g., N,N-dimethylaminocarbonyl) of hydroxy functional groups, esters groups (e.g. ethyl esters, morpholinoethanol esters) of carboxyl functional groups, N-acyl derivatives (e.g. N-acetyl) N-Mannich bases, Schiff bases and enaminones of amino functional groups, oximes, acetals, ketals and enol esters of ketone and aldehyde functional groups in compounds of Formula I, and the like, See Bundegaard, H. "Design of Prodrugs" p1-92, Elesevier, New York-Oxford (1985).

"Protecting group" refers to a grouping of atoms that when attached to a reactive group in a molecule masks, reduces or prevents that reactivity. Examples of protecting groups can be found in T.W. Green and P.G. Futs, Protective Groups in Organic Chemistry, (Wiley, 2^{nd} ed. 1991) and Harrison and Harrison et al., Compendium of Synthetic Organic Methods, Vols. 1-8 (John Wiley and Sons, 1971-1996). Representative amino protecting groups include, formyl, acetyl, trifluoroacetyl, benzyl, benzyloxycarbonyl (CBZ), *tert*-butoxycarbonyl (Boc), trimethyl silyl (TMS), 2-trimethylsilyl-ethanesulfonyl (SES), trityl and substituted trityl groups, allyloxycarbonyl, 9-fluorenylmethyloxycarbonyl (FMOC), nitro-veratryloxycarbonyl (NVOC), and the like. Representative hydroxy protecting groups include those where the hydroxy group is either acylated or alkylated such as benzyl, and trityl ethers as well as alkyl ethers, tetrahydropyranyl ethers, trialkylsilyl ethers and allyl ethers.

"Treating" or "treatment" of a disease includes: (1) preventing the disease, i.e., causing the clinical symptoms of the disease not to develop in a mammal that may be exposed to or predisposed to the disease but does not yet experience or display symptoms of the disease; (2) inhibiting the disease, i.e., arresting or reducing the development of the disease or its clinical symptoms; or (3) relieving the disease, i.e., causing regression of the disease or its clinical symptoms.

"A therapeutically effective amount" means the amount of a compound that, when administered to a mammal for treating a disease, is sufficient to effect such treatment for the disease. The "therapeutically effective amount" will vary depending on the compound, the disease and its severity and the age, weight, etc., of the mammal to be treated.

The term "treating", "contacting" or "reacting" when referring to a chemical reaction, means to add or mix two or more reagents under appropriate conditions to produce the indicated and/or the desired product. It should be appreciated that the reaction which produces the indicated and/or the desired product may not necessarily result directly from the combination of two reagents which were initially added, i.e., there can be one or more intermediates which are produced in the mixture which ultimately leads to the formation of the indicated and/or the desired product.

In case in the drawings of structural formula in the present description"N" is only shown with one or two bonds to the rest of the structure or "O" is only shown with one bond to the rest of the structure, the person skilled in the art will understand that in the case of "N" two or one "H"-atoms, respectively and in case of "O" one "H"-atom is/are present in the formula but not shown by the computer program used to draw the structures, e.g. ISIS draw. Therefore "-N" represents "-NH₂", "-N-" represents "-NH-" and "-O" represents "-OH".

One aspect of the present invention provides compounds represented by the formula: 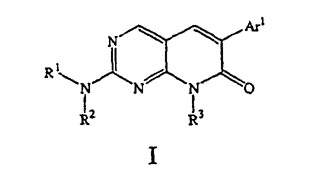 where:
R¹ is hydrogen or alkyl;
R² is -CR'R"-R^{a} (where R' and R" are hydrogen, hydroxyalkyl or alkyl with at least one being alkyl or hydroxyalkyl and R^{a} is hydroxyalkyl), R^{x}-S(O)₂-R^{y} (where R^{x} is alkyl and R^{y} is alkenyl), alkoxy-substituted alkyl, heterocyclylalkyl or C₄-C₅ cycloalkyl, wherein each of the hydroxy group present in R² can be independently in the form of an ester, a carbamate, a carbonate, or a sulfonate derivative; or
R¹ and R² together with the nitrogen atom to which they are attached form heterocyclyl;
R³ is hydrogen, alkyl, amino, monoalkylamino, dialkylamino, cycloalkyl, aryl, aralkyl, haloalkyl, heteroalkyl, cyanoalkyl, alkylene-C(O)-R (where R is hydrogen, alkyl, hydroxy, alkoxy, amino, monoalkylamino or dialkylamino) or acyl; and
Ar¹ is aryl.

Particularly preferred compounds of Formula I are those represented by the Formula II: 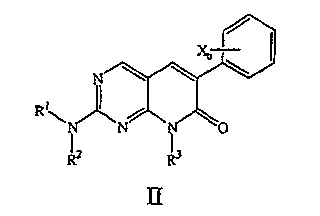 where n is 1 or 2, preferably 1, and X is hydrogen, alkyl, halo, nitro, cyano or methoxy, preferably halogene.

More preferred compounds of Formula I are those represented by the Formula III:

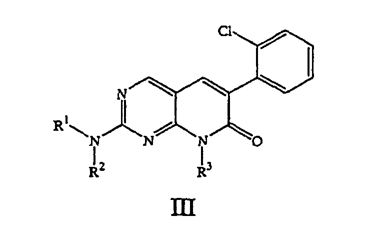

In reference to compounds of Formula I, II or III:

Preferably, R¹ is hydrogen or methyl. More preferably, R¹ is hydrogen.

Preferably, R² of compounds of Formula I is -CR'R"-R^{a} (where R' and R" are hydrogen, hydroxyalkyl or alkyl with at least one being alkyl or hydroxyalkyl and R^{a} is hydroxyalkyl), alkoxy-substituted alkyl, R^{x}-SO₂-R^{y} (where R^{x} is alkyl and R^{y} is alkenyl), C₄-C₅ cycloalkyl, or (N-substituted piperidin-4-yl)methyl, and more preferably - CR'R"-R^{a} (as defined before) or (N-substituted piperidin-4-yl)methyl, specifically (N-substituted piperidin-4-yl) methyl, whereby the substitution is preferably as indicated in the definitions before, wherein each of the hydroxy group present in R² can be independently in the form of an ester, a carbamate, a carbonate, or a sulfonate derivative. Even more preferably R² is (1,1-dimethyl-2-hydroxy)ethyl, (1,2-dimethyl-2-hydroxy)propyl, (N-methylpiperidin-4-yl)methyl, [1-dimethylacetamido-piperidin-4-yl]methyl, [1-carboxymethyl-piperidin-4-yl]methyl, (1,1-dimethyl-2-hydroxy)ethyl, (1-methyl-3-hydroxy)propyl, (1-methyl-1-hydroxymethyl-2-hydroxy)ethyl, [1,1-di(hydroxymethyl)]propyl, (1-hydroxymethyl-2-methyl)propyl, (1-hydroxymethyl)propyl, (1-hydroxymethyl-2,2-dimethyl)propyl, (1-hydroxymethyl-3-methyl)butyl, (2-hydroxy)propyl, (1-methyl-2-hydroxy)ethyl, (1-hydroxymethyl-2-methyl)butyl, 2-hydroxyethyl, 2-hydroxy-2-methylpropyl, 5-hydroxypentyl, 2-hydroxybutyl, 1-(hydroxymethyl)-2-hydroxyethyl, 2,3-dihydroxypropyl, 1-carbomethoxymethyl-piperidin-4-yl]methyl, or[1-carboxymethylpiperidin-4-yl]methyl, or {1-methyl, 2-methyl, 2-[methyl-disulfone]}-ethyl, wherein each of the hydroxy group present in R² can be independently in the form of an ester, a carbamate, a carbonate, or a sulfonate derivative.

In another embodiment, preferably R¹ and R² together with the nitrogen atom to which they are attached form -alkylene-S(O)ₙ-R^{a}-substituted heterocyclyl (where n is 0, 1 or 2, preferably 0, and R^{a} is alkyl). More preferably, R¹ and R² together with the nitrogen atom to which they are attached form -alkylene-S(O)ₙ-R^{a}- substituted aziridinyl, where n is preferably 0.

Preferably, R³ is hydrogen, alkyl, amino, monoalkylamino, dialkylamino, haloalkyl, cycloalkyl, cyanomethyl, heteroalkyl, aryl, aralkyl or alkylene-C(O)-R (where R is hydrogen, alkyl, hydroxy, alkoxy, amino, monoalkylamino or dialkylamino). More preferably R³ is hydrogen, alkyl, amino, cycloalkyl or aryl, especially hydrogen, alkyl or aryl. R³ can preferably also be hydrogen, amino, methyl, 2,2,2-trifluoroethyl, cyclopropyl, cyanomethyl, 2-hydroxyethyl, 4-fluorophenyl, benzyl, carboxymethyl or methoxycarbonylmethyl. Even more preferably R³ is hydrogen, methyl, 4-fluorophenyl, amino or cyclopropyl, especially hydrogen, methyl or 4-fluorophenyl.

In a further aspect the present invention provides compounds represented by the formula: 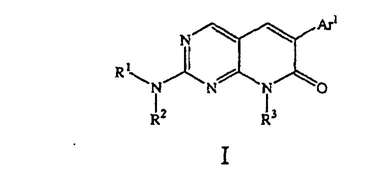 where:
R¹ is hydrogen or alkyl;
R² is -CR'R"-R^{a} (where R' and R" are hydrogen or alkyl with at least one being alkyl and R^{a} is hydroxyalkyl) or heterocyclylalkyl;
R³ is hydrogen, alkyl, cycloalkyl, aryl, aralkyl, haloalkyl, heteroalkyl, cyanoalkyl, alkylene-C(O)-R (where R is hydrogen, alkyl, hydroxy, alkoxy, amino, monoalkylamino or dialkylamino) or acyl; and
Ar¹ is aryl.

Particularly preferred compounds of this aspect of the present invention are those represented by the Formula II: 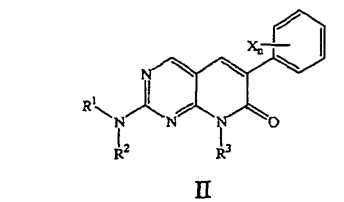 where n is 1 or 2, preferably 1, and X is hydrogen, alkyl, halo, nitro, cyano or methoxy, preferably halogene.

More preferred compounds of this aspect of the present invention are those represented by the Formula III:

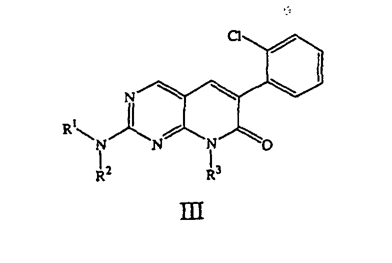

In reference to compounds of Formula I of this aspect of the present invention:
Preferably, R¹ is hydrogen or methyl. More preferably, R¹ is hydrogen.
Preferably, R² is -CR'R"-R^{a} (where R' and R" are hydrogen, or alkyl with at least one being alkyl and R^{a} is hydroxyalkyl;
or (N-substituted piperidin-4-yl)methyl, More preferably R² is (1,1-dimethyl-2-hydroxy)ethyl, (1,2-dimethyl-2-hydroxy)propyl, (N-methyl piperidin-4-yl)methyl, [1-dimethylacetamido-piperidin-4-yl]methyl, [1-carboxymethyl-piperidin-4-yl]methyl, or [1-carboxymethoxymethylpiperidin-4-yl]methyl.
Preferably, R³ is hydrogen alkyl, haloalkyl, cycloalkyl, cyanomethyl, heteroalkyl, aryl, aralkyl or alkylene-C(O)-R (where R is hydrogen, alkyl, hydroxy, alkoxy, amino, monoalkylamino or dialkylamino). Most preferably R³is hydrogen, methyl, 2,2,2-trifluoroethyl, cyclopropyl, cyanomethyl, 2-hydroxyethyl, 4-fluorophenyl, benzyl, carboxymethyl or methoxycarbonylmethyl. Even more preferably R³ is hydrogen or methyl.

In an even further aspect the present invention provides compounds of formula I, II or III as herein defined before wherein R² is heterocyclyalkyl and/or R³ is amino with the definitions and preferences for heterocyclylalkyl and amino, R¹ and Ar¹ as given hereinbefore.

It should be appreciated that when R³ is hydrogen, the compounds can exist in tautomeric form as follows:

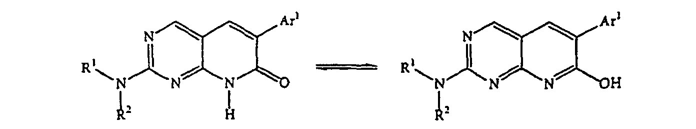

Thus, in addition to the compounds described above, the present invention includes all tautomeric forms. Furthermore, the present invention also includes all pharmaceutically acceptable salts of those compounds along with prodrug forms of the compounds and all stereoisomers whether in a pure chiral form or a racemic mixture or other form of mixture.

Still further, combinations of the preferred groups described above will form other preferred embodiments; thus, for example, preferred substituents R¹, R² and R³ of Formula I are also preferred substituents of compounds of Formulas II and III.

Some of the representative compounds of Formula I are shown in Table 1 below.

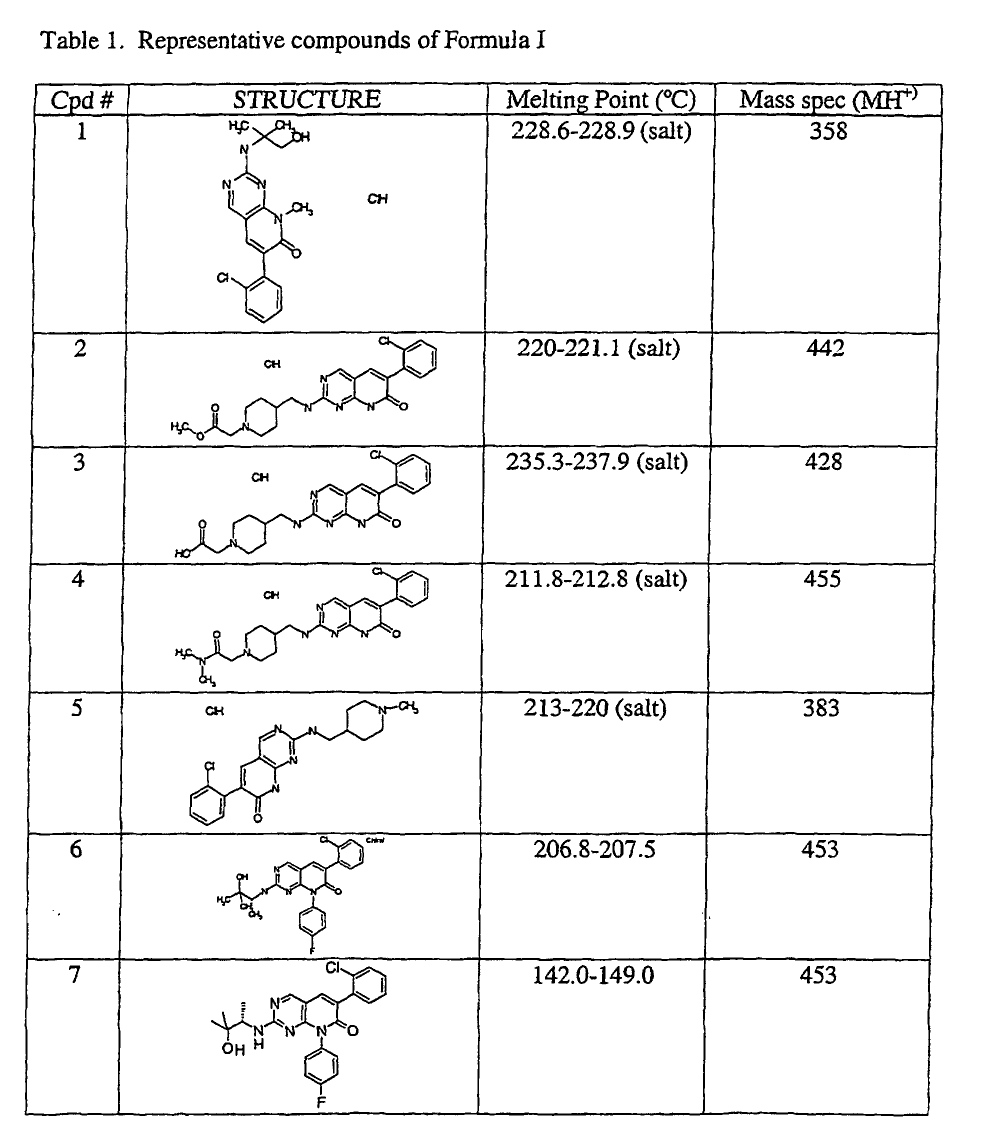

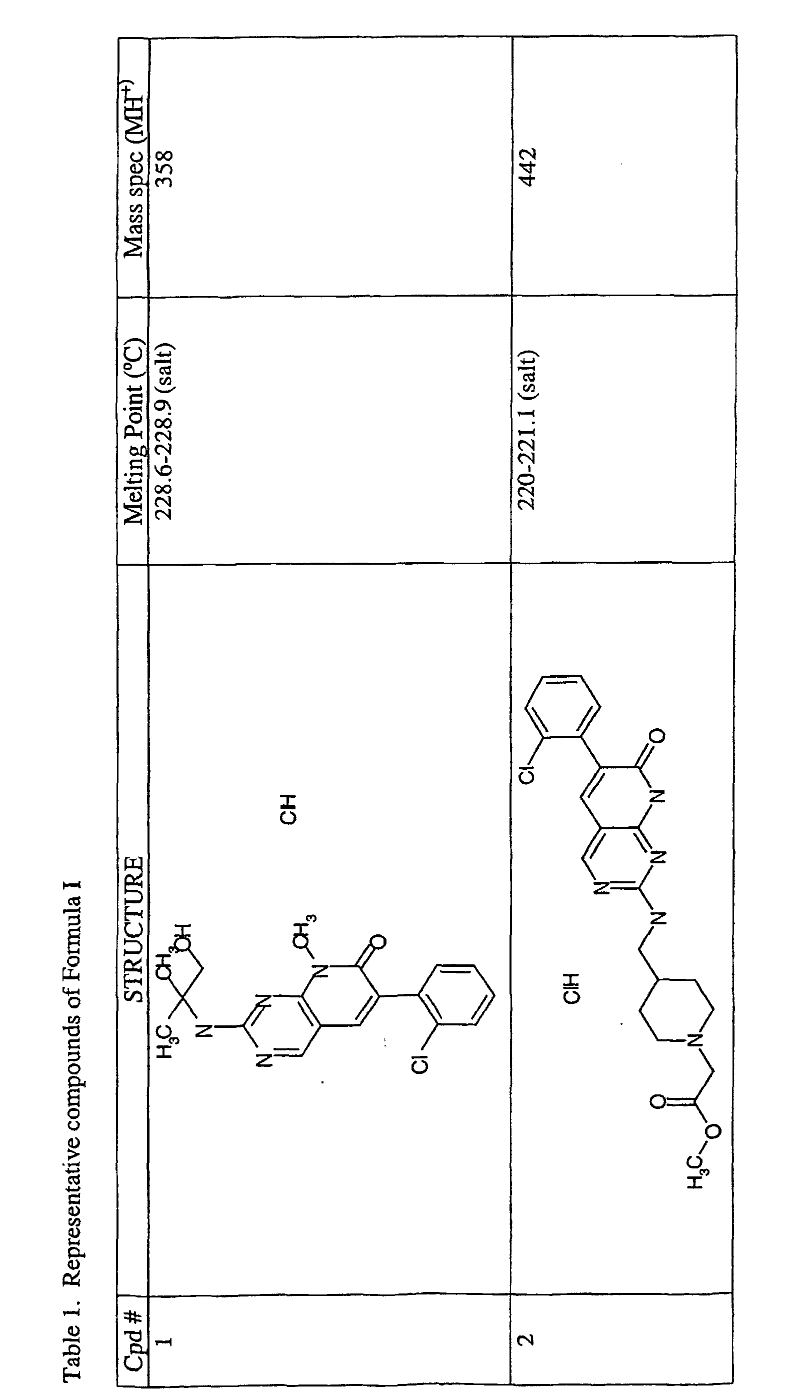

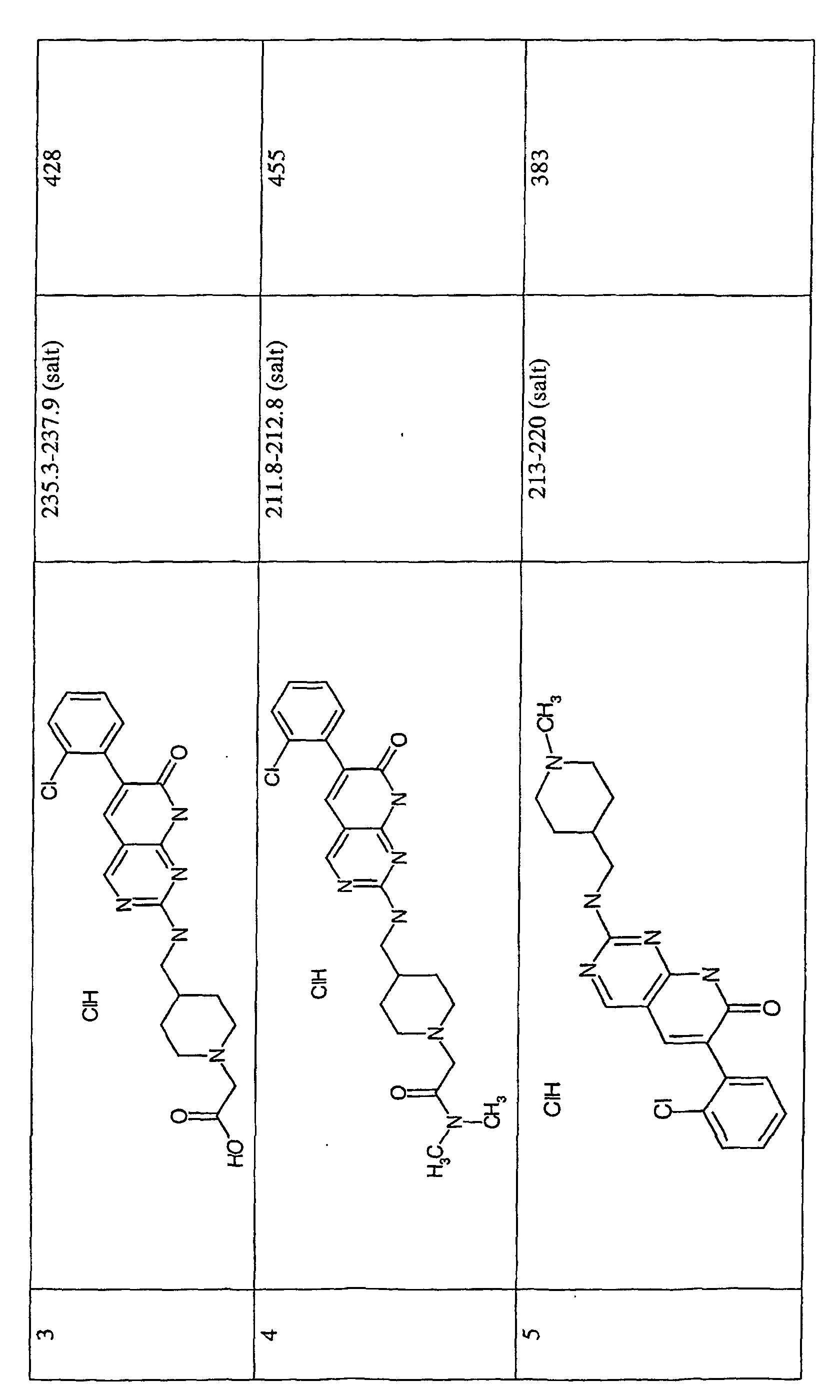

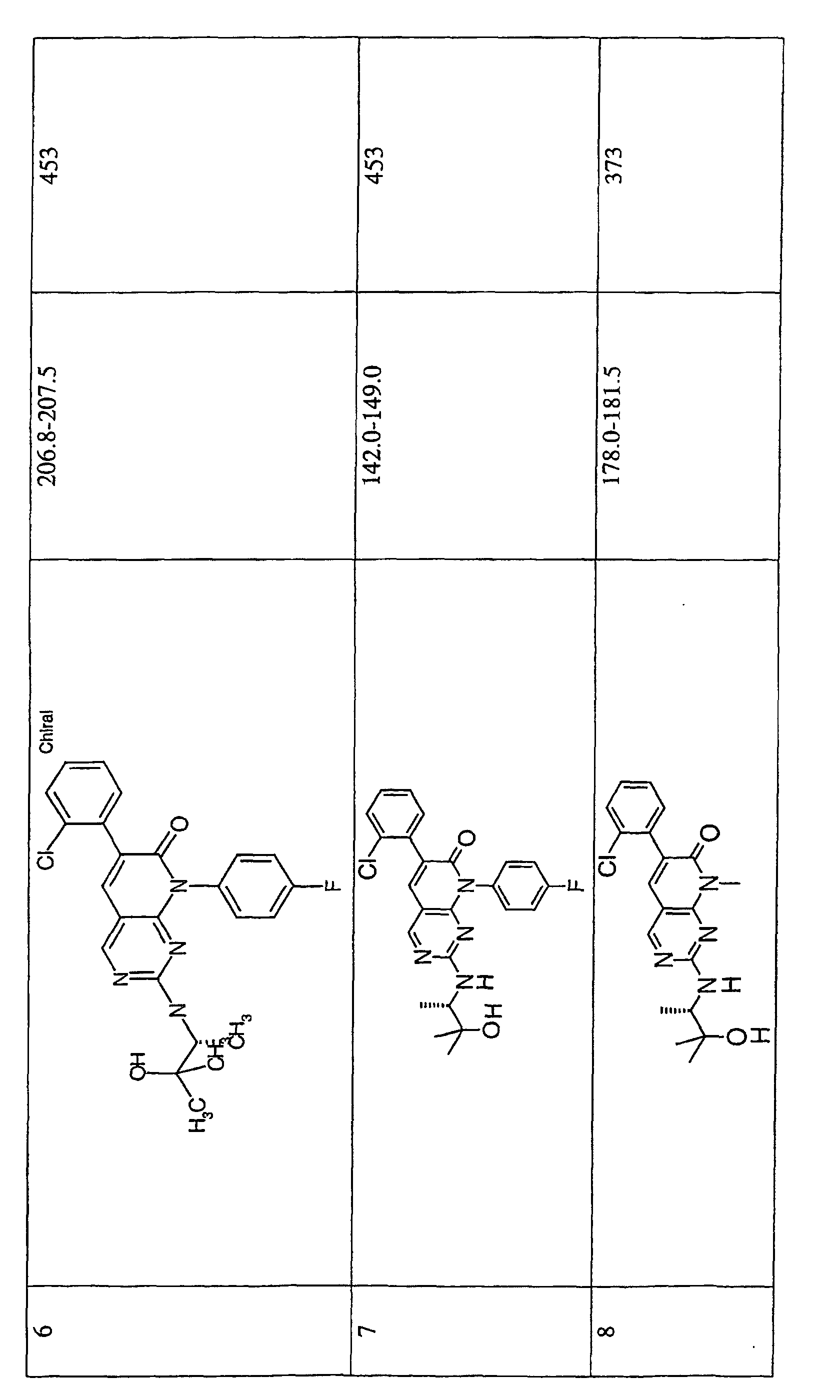

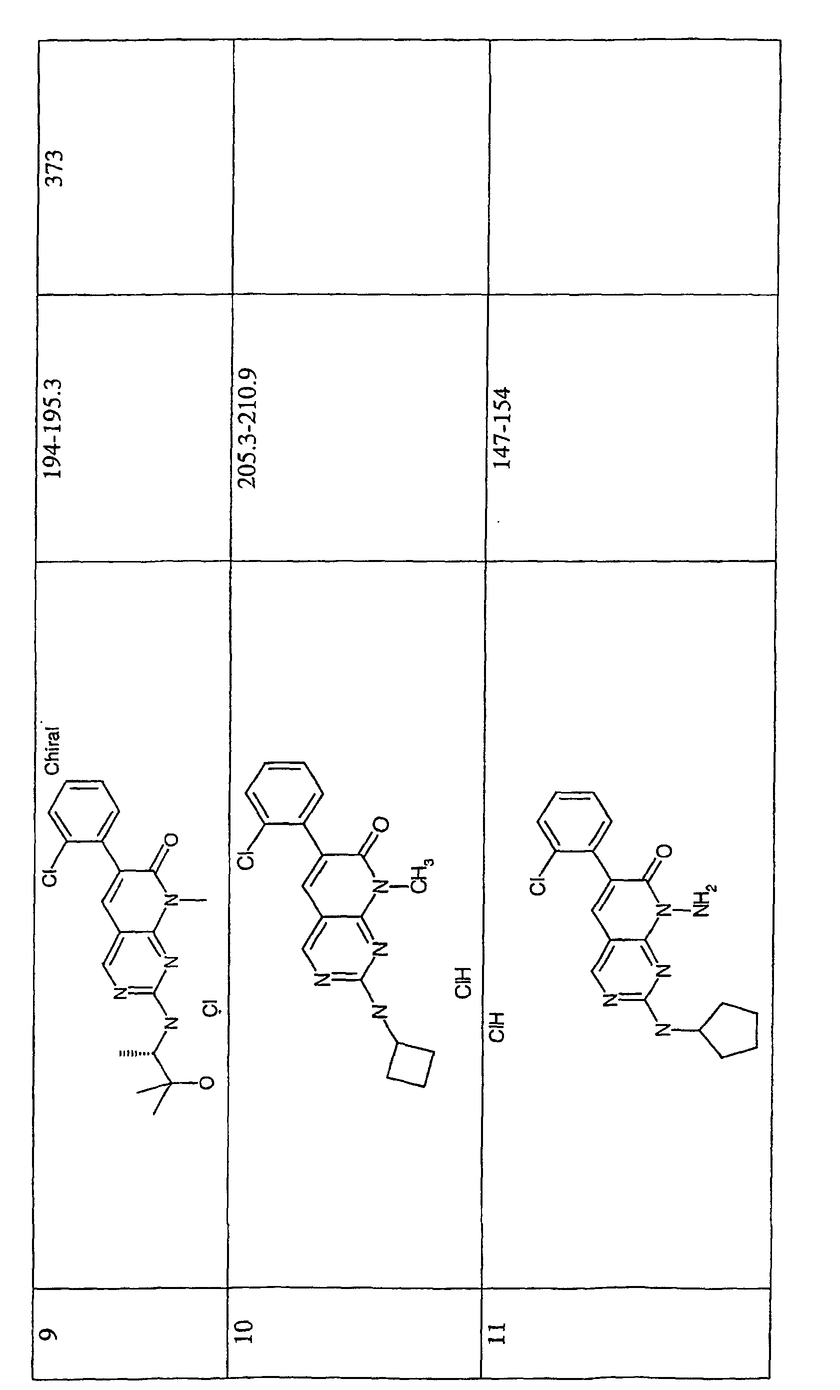

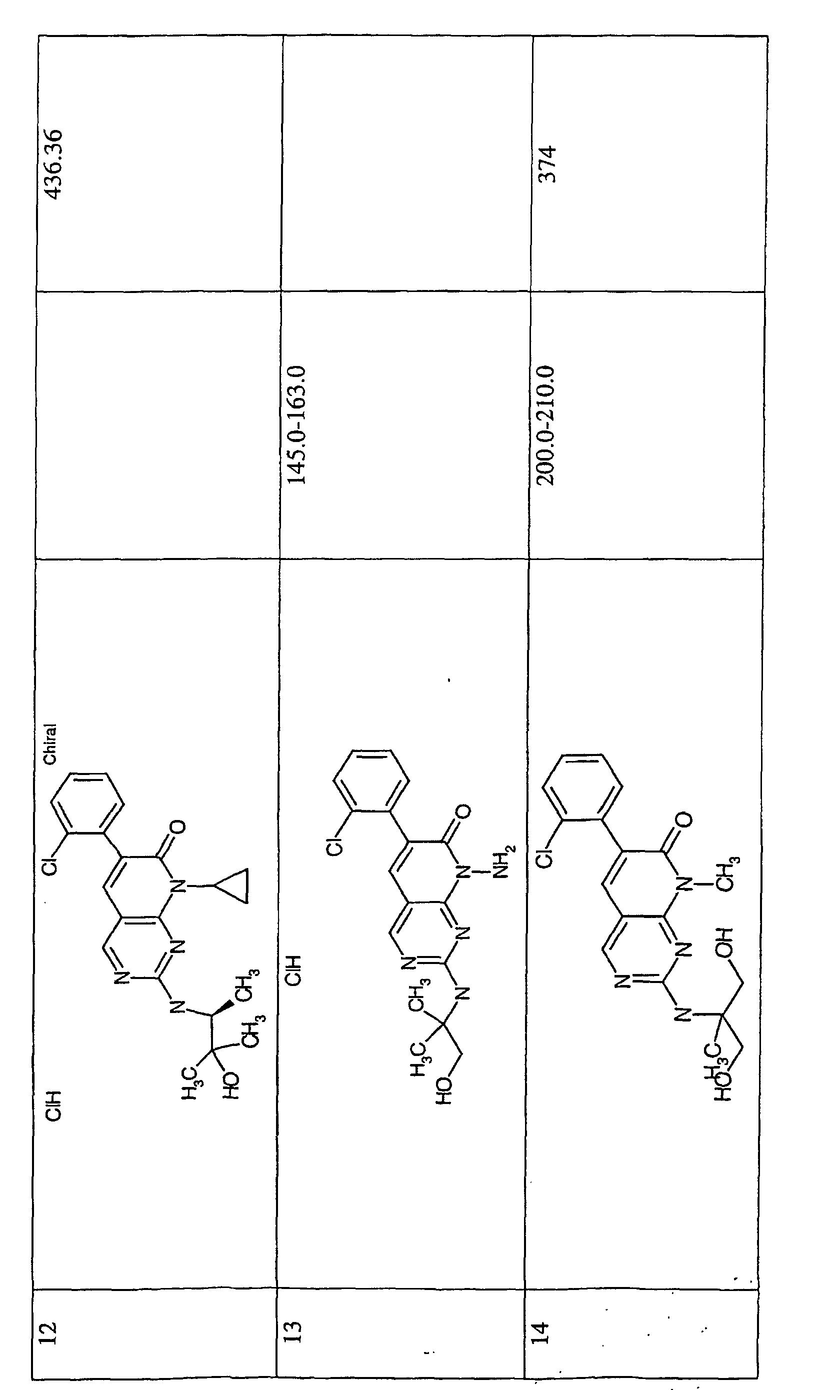

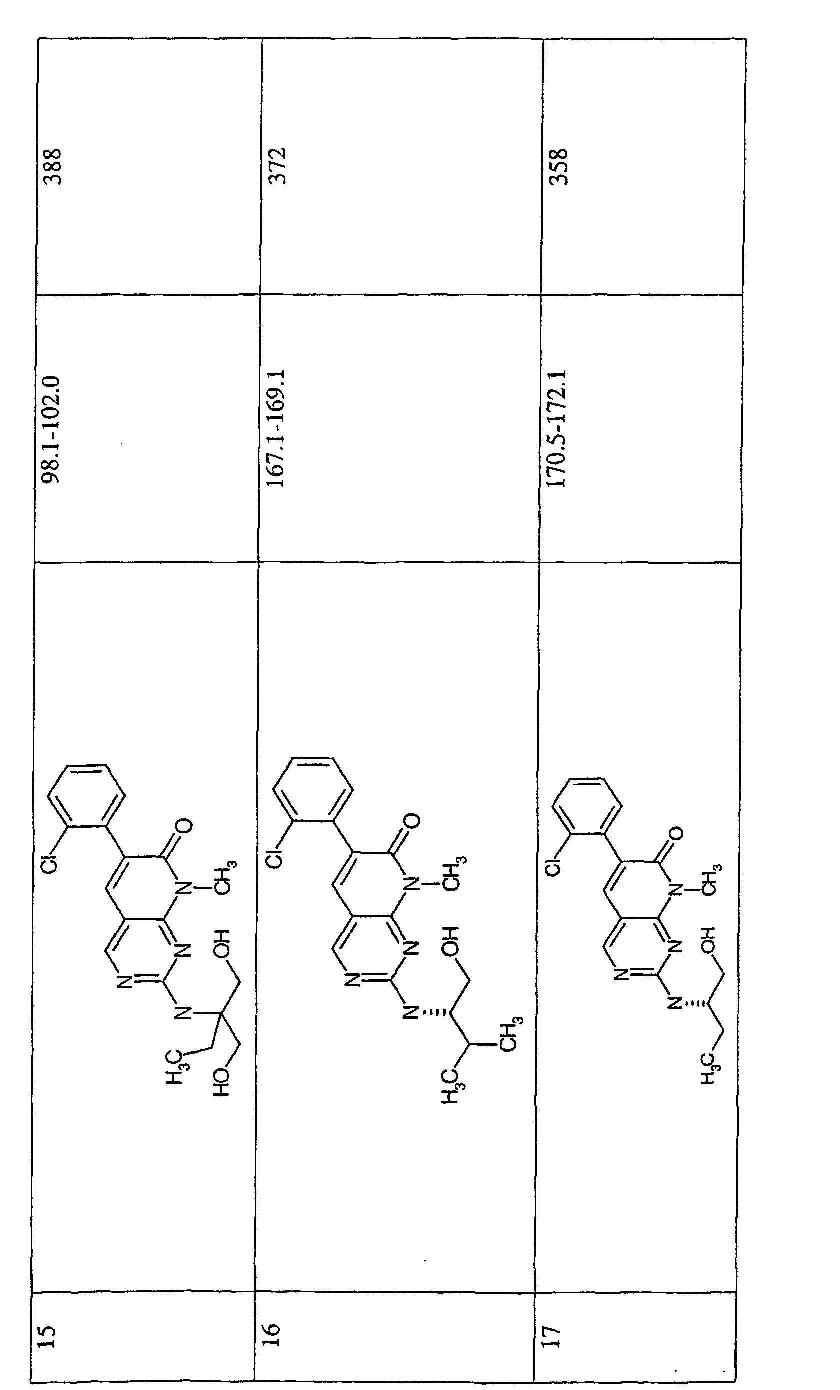

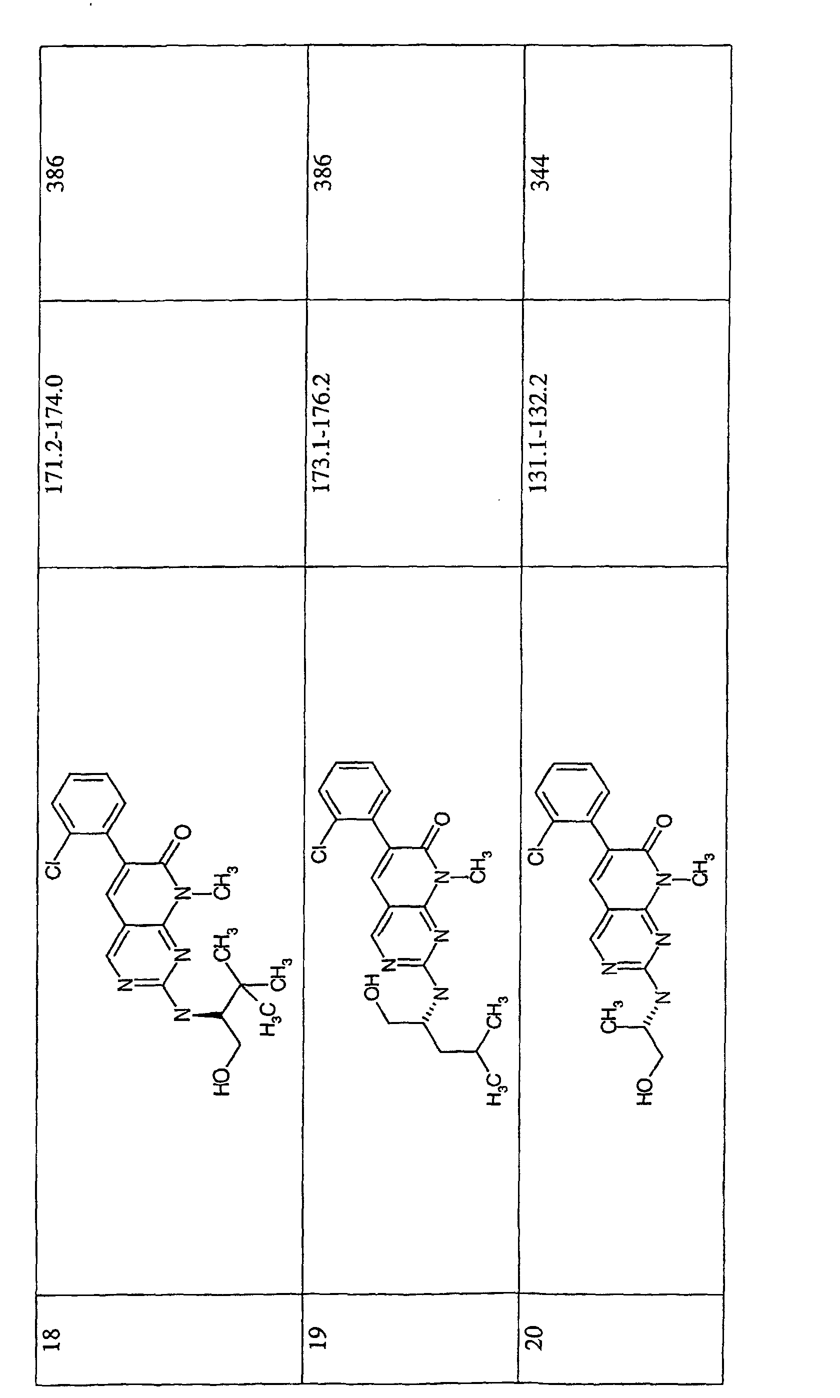

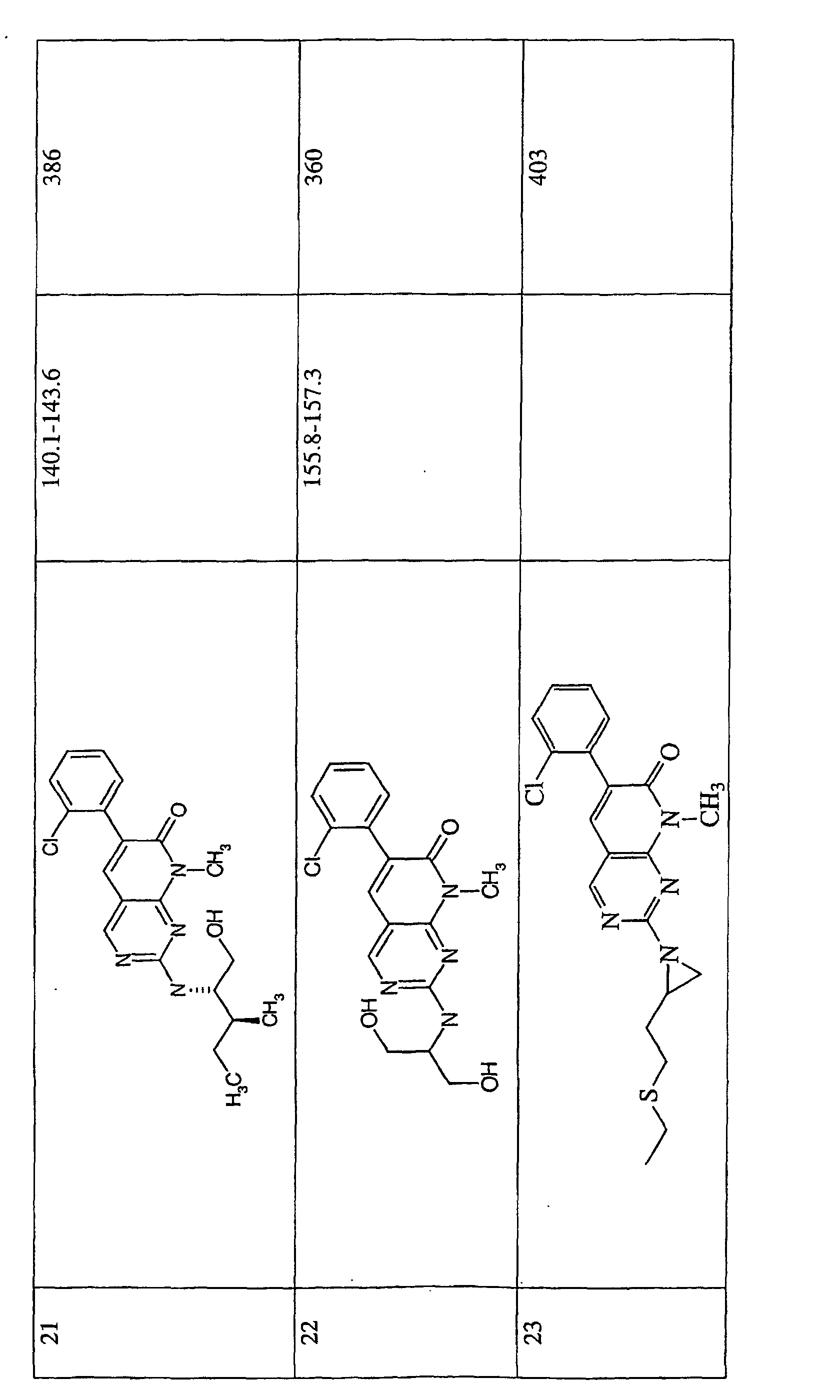

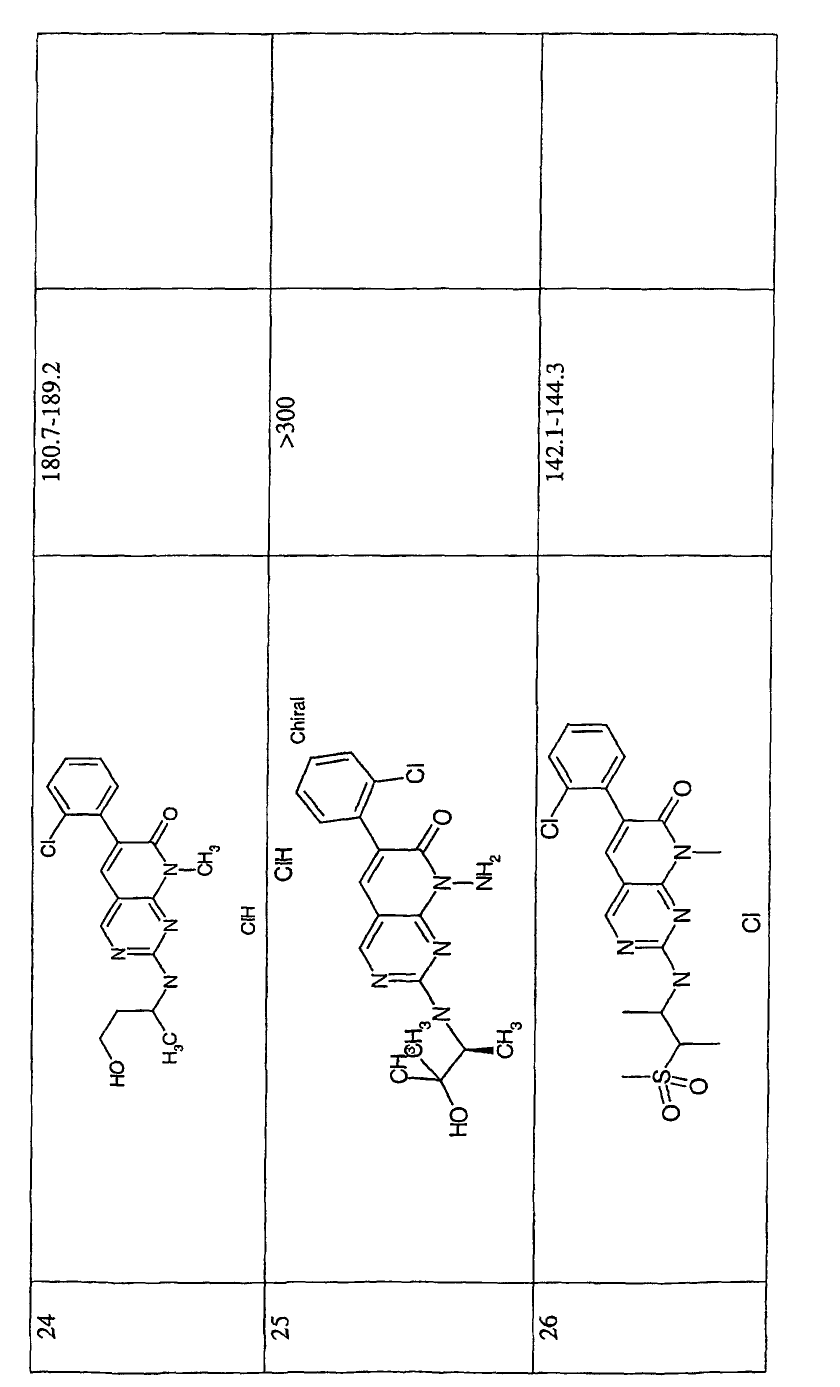

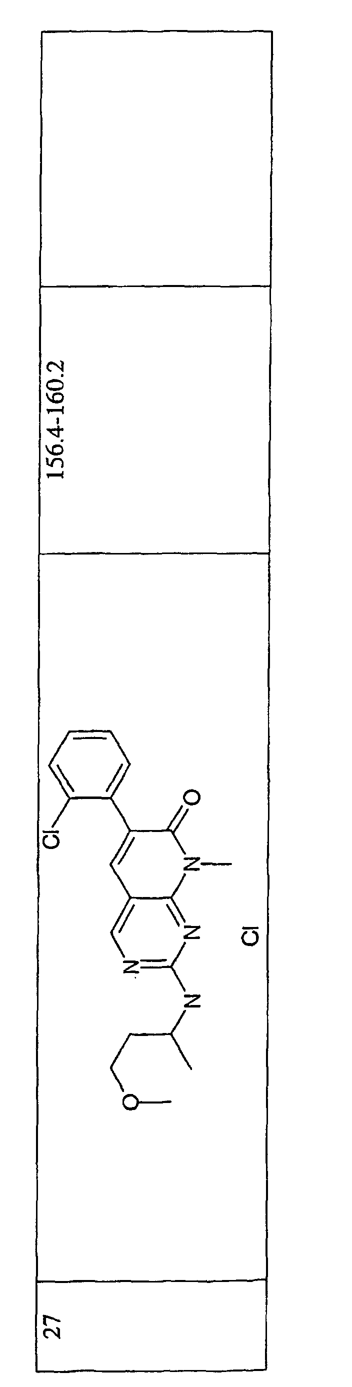

The IC₅₀ of Compounds of Formula I in the *in vitro* p38 assay is less than 10 µM, preferably less than 5 µM, more preferably less than 2 µM, and most preferably less than 1 µM. In particular, Compounds of Formula I in Table I have IC₅₀ in the *in vitro* p38 assay of from about 0.712 µM to about 0.001 µM.

The compounds of the present invention can exist in unsolvated forms as well as solvated forms, including hydrated forms. In general, the solvated forms, including hydrated forms, are equivalent to unsolvated forms and are intended to be encompassed within the scope of the present invention. Furthermore, as stated above, the present invention also includes all pharmaceutically acceptable salts of those compounds along with prodrug forms of the compounds and all stereoisomers whether in a pure chiral form or a racemic mixture or other form of mixture.

The compounds of the present invention are capable of further forming pharmaceutically acceptable acid addition salts. All of these forms are within the scope of the present invention.

Pharmaceutically acceptable acid addition salts of the compounds of the present invetion include salts derived from inorganic acids such as hydrochloric, nitric, phosphoric, sulfuric, hydrobromic, hydroiodic, phosphorous, and the like, as well as the salts derived from organic acids, such as aliphatic mono- and dicarboxylic acids, phenyl-substituted alkanoic acids, hydroxy alkanoic acids, alkanedioic acids, aromatic acids, aliphatic and aromatic sulfonic acids, etc. Such salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate, and the like. Also contemplated are salts of amino acids such as arginate and the like and gluconate, galacturonate (see, for example, Berge et al., "Pharmaceutical Salts," *J. of Pharmaceutical Science,* 1977, *66*, 1-19).

The acid addition salts of the basic compounds can be prepared by contacting the free base form with a sufficient amount of the desired acid to produce the salt in the conventional manner. The free base form can be regenerated by contacting the salt form with a base and isolating the free base in the conventional manner. The free base forms may differ from their respective salt forms somewhat in certain physical properties such as solubility in polar solvents, but otherwise the salts are equivalent to their respective free base for purposes of the present invention.

Pharmaceutically acceptable base addition salts can be formed with metal ions or amines, such as alkali and alkaline earth metal ions or organic amines. Examples of metal ions which are used as cations include sodium, potassium, magnesium, calcium, and the like. Examples of suitable amines are N,N'-dibenzylethylenediamine, chloroprocaine, choline, diethanolamine, ethylenediamine, N-methylglucamine, and procaine (see, for example, Berge et al , "Pharmaceutical Salts," *J. of Pharmaceutical Science,* 1977, *66*, 1-19).

The base addition salts of acidic compounds can be prepared by contacting the free acid form with a sufficient amount of the desired base to produce the salt in the conventional manner. The free acid form can be regenerated by contacting the salt form with an acid and isolating the free acid in the conventional manner. The free acid forms may differ from their respective salt forms somewhat in certain physical properties such as solubility in polar solvents, but otherwise the salts are equivalent to their respective free acid for purposes of the present invention.

The compounds of the present invention can be prepared by a variety of methods, using procedures well known to those of skill in the art. In one aspect of the present invention, a method for preparing compounds of Formula I is shown in Scheme 1 below.

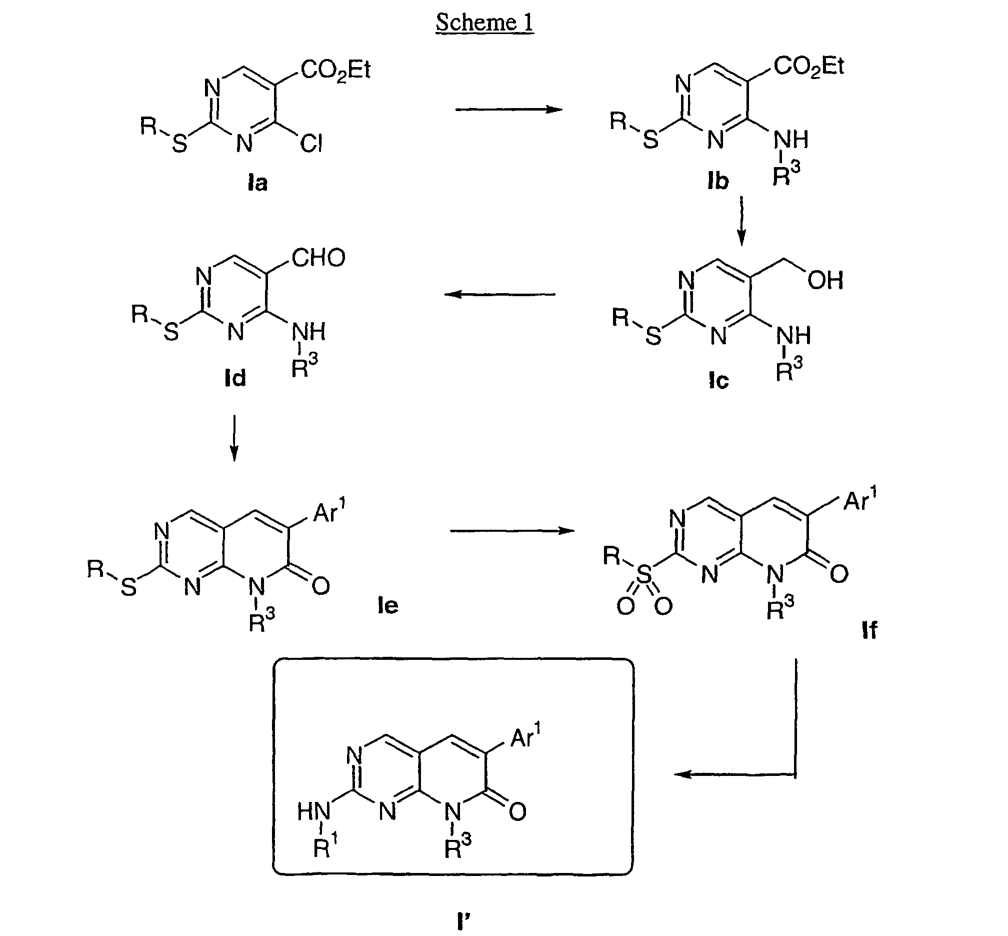

Treatment of a compound of Formula Ia (can be easily prepared by methods known in the art or can be purchased from sources known to the man skilled in the art) with a primary amine (R³-NH₂) provides a compound of Formula Ib. This reaction is conveniently carried out in a solvent which is inert under the reaction conditions, preferably a halogenated aliphatic hydrocarbon, especially dichloromethane, an optionally halogenated aromatic hydrocarbon, an open-chain or cyclic ether (e.g. tetrahydrofuran), a formamide or a lower alkanol. Suitably, the reaction is carried out at about -20 °C to about 120 °C.

Reduction of a compound of Formula Ib provides an alcohol of Formula Ic. This reduction is typically carried out using lithium aluminum hydride in a manner well known to those of skill in the art (e.g., in a solvent which is inert under the conditions of the reduction, preferably an open-chain or cyclic ether, especially tetrahydrofuran, at about -20 °C to about 70 °C, preferably at about 0 °C to about room temperature).

Oxidation of an alcohol of Formula Ic in the next step provides a carboxaldehyde of Formula Id. The oxidation is typically carried out with manganese dioxide, although numerous other methods can also be employed (see, for example, Advanced Organic Chemistry, 4^{TH} Ed., March, John Wiley & Sons, New York (1992)). Depending on the oxidizing agent employed, the reaction is carried out conveniently in a solvent which is inert under the specific oxidation conditions, preferably a halogenated aliphatic hydrocarbon, especially dichloromethane, or an optionally halogenated aromatic hydrocarbon. Suitably, the oxidation is carried out at about 0 °C to about 60 °C.

Reaction of a carboxaldehyde of Formula Id with an aryl substituted acetate Ar¹-CH₂-CO₂R (where R is an alkyl group) in a presence of a base provides a compound of Formula Ie. Any relatively non-nucleophilic base can be used including carbonates, such as potassium carbonate, lithium carbonate, and sodium carbonate; bicarbonates, such as potassium bicarbonate, lithium bicarbonate, and sodium bicarbonate; amines, such as secondary and tertiary amines; and resin bound amines such as 1,3,4,6,7,8-hexahydro-2H pyrimido[1,2-a]pyrimidine. To increase the product yield and/or to increase the reaction rate, water which is formed in the reaction can be removed by azeotrope. Conveniently, the reaction is carried out in a solvent which is relatively polar but inert under the reaction conditions, preferably an amide such as dimethyl formamide, N-substituted pyrrolidinone, especially 1-methyl-2-pyrrolidinone, and at a temperature of about 70 °C to about 150 °C, especially at or near the reflux temperature of the solvent to assist in the noted azeotropic removal of water.

Oxidation of Ie with an oxidizing agent, such as 3-chloroperbenzoic acid (i.e., MCPBA) and Oxone® provides a sulfone (If) which can be converted to a variety of target compounds. Typically the oxidation of Ie is carried out in a solvent which is inert under the conditions of the oxidation. For example, when MCPBA is used as the oxidizing agent, the solvent is preferably a halogenated aliphatic hydrocarbon, especially chloroform. When Oxone® is used as the oxidizing agent, the solvent can be water or a mixture of an organic solvent (such as acetonitrile) and water. The reaction temperature depends on the solvent used. For an organic solvent, the reaction temperature is generally at about -20 °C to about 50 °C, preferably about 0 °C to about room temperature. When water is used as the solvent, the reaction temperature is generally from about 0 °C to about 50 °C, preferably about 0 °C to about room temperature. Alternatively, the oxidation can be carried under catalytic conditions with rhenium/peroxide based reagents. See, for example, "Oxidation of Sulfoxides by Hydrogen Peroxide, Catalyzed by Methyltrioxorhenium(VII)", Lahti, David W.; Espenson, James H, *Inorg. Chem*. 2000, *39*(10) pp.2164-2167; "Rhenium oxo complexes in catalytic oxidations," *Catal. Today,* 2000, *55*(4), pp317-363 and "A Simple and Efficient Method for the Preparation of Pyridine N-Oxides", Coperet, Christophe; Adolfsson, Hans; Khuong, Tinh-Alfredo V.; Yudin, Andrei K.; Sharpless, K. Barry, J. *Org. Chem*., 1998, *63*(5), pp1740-1741, which are incorporated herein by reference in their entirety.

Reaction of the compound If with an amine (R²-NH₂) provides the compounds of Formula I' (i.e. compounds I, wherein R¹ is hydrogen). Further alkylation of I' then provides compounds of Formula I, where R¹ is not hydrogen. The reaction can be carried out in the presence or absence of solvent. Conveniently, the reaction is carried out at temperatures of from about 0 °C to about 200 °C, more preferably about room temperature to about 150 °C. Alternatively, in some cases rather than using the sulfone If, the sulfide Ie or the corresponding sulfoxide can be reacted directly an amine (R¹-NH₂) to provide the compounds of Formula I'. Furthermore, If can also be alkylated with an amine of R¹R²NH directly to provide a compound of Formula I where R¹ and R² are as described in the Summary of the Invention.

Accordingly, the present invention provides a method of preparing compounds of Formula I, by treating a compound of general Formula Ie or If with an amine (R¹-NH₂) and optionally reacting the resulting product with R¹-L, where R¹ is defined above, but excludes hydrogen, and L is a leaving group.

Alternatively, the carboxaldehyde of the Compound of Formula Ie can be prepared as shown in Scheme II below, which eliminates a need for an ester reduction and an alcohol oxidation in Scheme I.

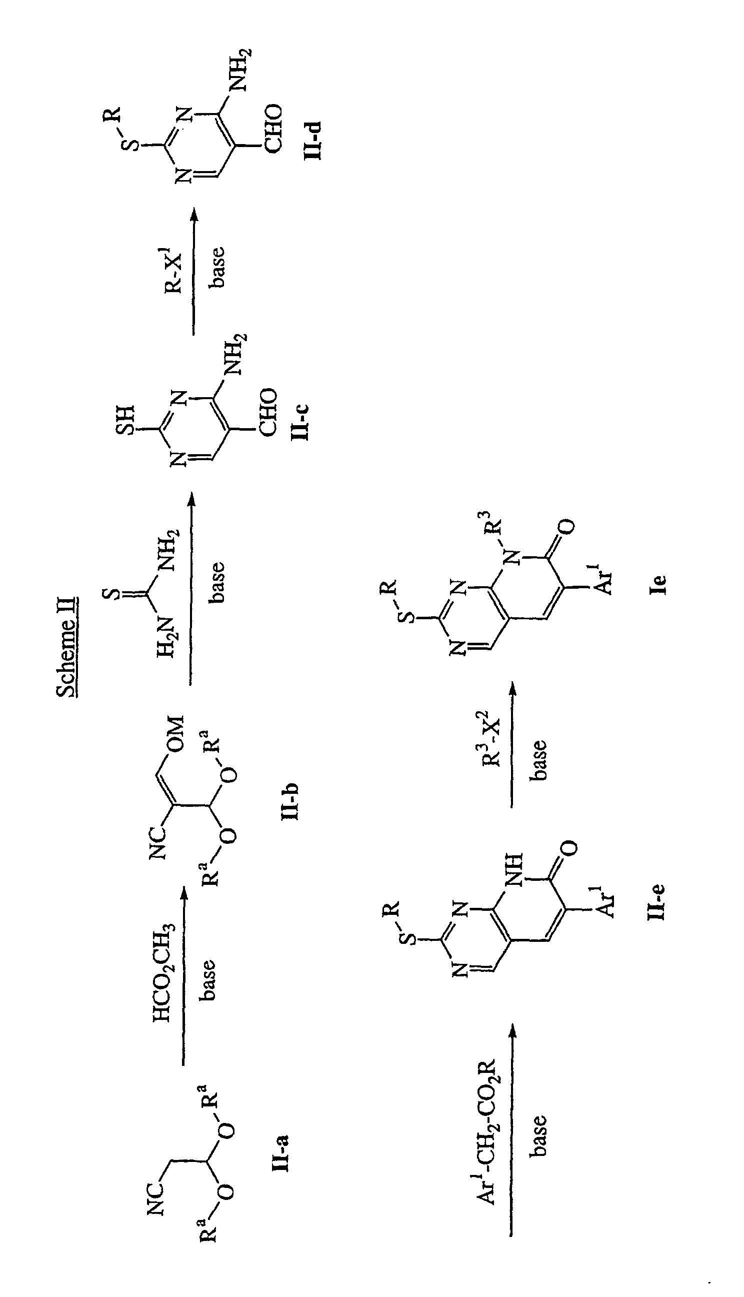

Treatment of a compound of Formula II-a (where each R^{a} is independently alkyl) (can be easily prepared by methods known in the art or can be purchased from sources known to the man skilled in the art) with an alkyl formate (e.g., methylformate) in the presence of a base provides a compound of Formula II-b (where M is a metal). This reaction is conveniently carried out at a temperature range of from about 0 °C to about 100 °C. Typically, an ether, such as THF, and other solvents which are inert to the reaction conditions is used. Suitable bases include alkoxides, such as tert-butoxides, and other relatively non-nucleophilic bases that are capable of deprotonating the compound of Formula II-a.

Cyclization of a compound of Formula II-b with thiourea in the presence of a base affords a pyrimidine of Formula II-c. Typically, this cyclization reaction is conducted in an alcoholic solvent under refluxing conditions using a corresponding alkoxide as a base.

Alkylation of a compound of Formula II-c with an alkylating agent R-X¹ (where R is an alkyl group and X¹ is a leaving group, such as halide) in the presence of a base then provides a compound of Formula II-d. Suitable bases include a relatively non-nucleophilic bases including carbonates, such as potassium carbonate, lithium carbonate, and sodium carbonate; and bicarbonates, such as potassium bicarbonate, lithium bicarbonate, and sodium bicarbonate. Conveniently, the reaction is carried out in a relatively polar solvent that inert under the reaction conditions, preferably acetone, dimethylformamide (DMF) or methylpyrrolidinone (MP).

Reaction of a compound of Formula II-d with an aryl substituted acetate Ar¹-CH₂-CO₂R (where R is an alkyl group) under similar conditions as that described for preparation of a compound of Formula Ie in Scheme I above, then provides a compound of Formula II-e. While the alkylation of a compound of Formula II-c is generally conducted prior to the reaction with an aryl substituted acetate, the order of these two reactions are not crucial and can be reversed. Thus, a compound of Formula II-c can be reacted with an aryl substituted acetate Ar¹-CH₂-CO₂R and the resulting product can be alkylated with an alkylating agent R-X¹ to provide a compound of Formula II-e.

Alkylation of the amine group of a compound of Formula II-e with an alkylating agent R³-X² (where R³ is those defined above and X² is a leaving group, such as halide) then provides a compoud of Ie which can be further converted to a compound of Formula I' as described in Scheme I.

Thus, another aspect of the present invention provides a method of preparing a pyrimidine compound of Formula II-c by reacting an acetal of the Formula II-a with with an alkyl formate and reacting the resulting product with a thiourea.

Yet another aspect of the present invention provides a method for preparing a compound of Formula II-e, by reacting a compound of Formula II-c with an alkylating agent or an aryl substituted acetate, and reacting the resulting product with an aryl substituted acetate or an alkylating agent, respectively.

One of skill in the art will understand that certain modifications to the above schemes are contemplated and within the scope of the present invention. For example, certain steps will involve the use of protecting groups for functional groups that are not compatible with particular reaction conditions.

The compounds of the present invention can be used as medicaments, e.g., in the form of pharmaceutical preparations. The pharmaceutical preparations can be administered enterally, e.g., orally in the form of tablets, coated tablets, dragées, hard and soft gelatine capsules, solutions, emulsions or suspensions, nasally, e.g., in the form of nasal sprays, or rectally, e.g., in the form of suppositories. However, they may also be administered parenterally, *e*.*g*., in the form of injection solutions.

The compounds of Formula I and their aforementioned pharmaceutically acceptable salts can be processed with pharmaceutically inert, organic or inorganic carriers for the production of pharmaceutical preparations. Lactose, corn starch or derivatives thereof, talc, stearic acid or its salts and the like can be used, for example, as such carriers for tablets, coated tablets, dragées and hard gelatine capsules. Suitable carriers for soft gelatine capsules are, for example, vegetable oils, waxes, fats, semi-solid and liquid polyols and the like; depending on the nature of the active ingredient no carriers are, however, usually required in the case of soft gelatine capsules. Suitable carriers for the production of solutions and syrups are, for example, water, polyols, sucrose, invert sugar, glucose and the like. Suitable carriers for suppositories are, for example, natural or hardened oils, waxes, fats, semi-liquid or liquid polyols and the like.

The pharmaceutical preparations can also contain preservatives, solubilizers, stabilizers, wetting agents, emulsifiers, sweeteners, colorants, flavorants, salts for varying the osmotic pressure, buffers, masking agents or antioxidants. They can also contain therapeutically valuable substances other than the compounds of Formula I and their aforementioned pharmaceutically acceptable salts.

Medicaments which contain a compound of the present invention in association with a compatible pharmaceutical carrier material are also an object of the present invention, as is a process for the production of such medicaments which comprises bringing one or more of these compounds or salts and, if desired, one or more other therapeutically valuable substances into a galenical administration form together with a compatible pharmaceutical carrier.

As mentioned earlier, the compounds of the present invention can be used in accordance with the invention as therapeutically active substances, especially as antiinflammatory agents or for the prevention of graft rejection following transplant surgery. The dosage can vary within wide limits and will, of course, be fitted to the individual requirements in each particular case. In general, in the case of administration to adults a convenient daily dosage should be about 0.1 mg/kg to about 100 mg/kg, preferably about 0.5 mg/kg to about 5 mg/kg. The daily dosage may be administered as a single dose or in divided doses and, in addition, the upper dosage limit referred to earlier may be exceeded when this is found to be indicated.

Finally, the use of compounds of the present invention for the production of medicaments, especially in the treatment or prophylaxis of inflammatory, immunological, oncological, bronchopulmonary, dermatological and cardiovascular disorders, in the treatment of asthma, central nervous system disorders or diabetic complications or for the prevention of graft rejection following transplant surgery, is also an object of the invention.

Compounds of the present invention would be useful for, but not limited to, the treatment of any disorder or disease state in a human, or other mammal, which is exacerbated or caused by excessive or unregulated TNF or p38 kinase production by such mammal. Accordingly, the present invention provides a method of treating a cytokine-mediated disease which comprises administering an effective cytokine-interfering amount of a compound of the present invention.

Compounds of the present invention would be useful for, but not limited to, the treatment of inflammation in a subject, and for use as antipyretics for the treatment of fever. Compounds of the invention would be useful to treat arthritis, including but not limited to, rheumatoid arthritis, spondyloarthropathies, gouty arthritis, osteoarthritis, systemic lupus erythematosus and juvenile arthritis, osteoarthritis, gouty arthritis and other arthritic conditions. Such compounds would be useful for the treatment of pulmonary disorders or lung inflammation, including adult respiratory distress syndrome, pulmonary sarcoidosis, asthma, silicosis, and chronic pulmonary inflammatory disease. The compounds are also useful for the treatment of viral and bacterial infections, including sepsis, septic shock, gram negative sepsis, malaria, meningitis, cachexia secondary to infection or malignancy, cachexia secondary to acquired immune deficiency syndrome (AIDS), AIDS, ARC (AIDS related complex), pneumonia, and herpes virus. The compounds are also useful for the treatment of bone resorption diseases, such as osteoporosis, endotoxic shock, toxic shock syndrome, reperfusion injury, autoimmune disease including graft vs. host reaction and allograft rejections, cardiovascular diseases including atherosclerosis, thrombosis, congestive heart failure, and cardiac reperfusion injury, renal reperfusion injury, liver disease and nephritis, and myalgias due to infection.

The compounds are also useful for the treatment of Alzheimer's disease, influenza, multiple sclerosis, cancer, diabetes, systemic lupus erthrematosis (SLE), skin-related conditions such as psoriasis, eczema, burns, dermatitis, keloid formation, and scar tissue formation. Compounds of the invention also would be useful to treat gastrointestinal conditions such as inflammatory bowel disease, Crohn's disease, gastritis, irritable bowel syndrome and ulcerative colitis. The compounds would also be useful in the treatment of ophthalmic diseases, such as retinitis, retinopathies, uveitis, ocular photophobia, and of acute injury to the eye tissue. Compounds of the invention also would be useful for treatment of angiogenesis, including neoplasia; metastasis; ophthalmological conditions such as corneal graft rejection, ocular neovascularization, retinal neovascularization including neovascularization following injury or infection, diabetic retinopathy, retrolental fibroplasia and neovascular glaucoma; ulcerative diseases such as gastric ulcer; pathological, but non-malignant, conditions such as hemangiomas, including infantile hemangiomas, angiofibroma of the nasopharynx and avascular necrosis of bone; diabetic nephropathy and cardiomyopathy; and disorders of the female reproductive system such as endometriosis. The compounds of the invention may also be useful for preventing the production of cyclooxygenase-2.

Besides being useful for human treatment, these compounds are also useful for veterinary treatment of companion animals, exotic animals and farm animals, including mammals, rodents, and the like. More preferred animals include horses, dogs, and cats.

The present compounds may also be used in co-therapies, partially or completely, in place of other conventional antiinflammatories, such as together with steroids, cyclooxygenase-2 inhibitors, NSAIDs, DMARDS, immunosuppressive agents, 5-lipoxygenase inhibitors, LTB₄ antagonists and LTA₄ hydrolase inhibitors.

As used herein, the term "TNF mediated disorder" refers to any and all disorders and disease states in which TNF plays a role, either by control of TNF itself, or by TNF causing another monokine to be released, such as but not limited to IL-1, IL-6 or IL-8. A disease state in which, for instance, EL-1 is a major component, and whose production or action, is exacerbated or secreted in response to TNF, would therefore be considered a disorder mediated by TNF.

As used herein, the term "p38 mediated disorder" refers to any and all disorders and disease states in which p38 plays a role, either by control of p38 itself, or by p38 causing another factor to be released, such as but not limited to IL-3, IL-6 or IL-8. A disease state in which, for instance, IL-1 is a major component, and whose production or action, is exacerbated or secreted in response to p38, would therefore be considered a disorder mediated by p38.

As TNF-β has close structural homology with TNF-α (also known as cachectin), and since each induces similar biologic responses and binds to the same cellular receptor, the synthesis of both TNF-α and TNF-β are inhibited by the compounds of the present invention and thus are herein referred to collectively as "TNF" unless specifically delineated otherwise.

### Examples:

### Example 1:

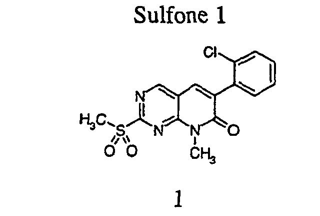

This example illustrates the preparation of sulfone 1 from ethyl 4-chloro-2-methylthiopyrimidine-5-carboxylate.

### Step 1.1 Preparation of ethyl 4-methylamino-2-methylthiopyrimidine-5-carboxylate

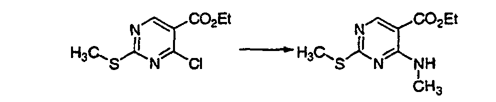

A solution of 20 g (86 mmol) of ethyl 4-chloro-2-methylthiopyrimidine-5-carboxylate (Aldrich Chemical Co., Milwaukee, Wisconsin, USA) in 250 mL of dichloromethane was cooled to 0 °C and treated slowly with 35 mL (281 mmol) of a 33% solution of methylamine in ethanol. After stirring for 30 minutes, 150 mL of water was added and the phases were separated. The organic phase was dried over magnesium sulfate and filtered. The filtrate was evaporated under reduced pressure to give 19 g (97%) of ethyl 4-methylamino-2-methylthiopyrimidine-5-carboxylate as a white solid.

### Step 1.2 Preparation of 4-methylamino-2-methylthiopyrimidine-5-methanol

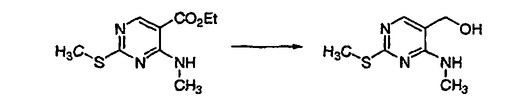

Lithium aluminum hydride (9 g, 237 mmol) was stirred in 300 mL of dry tetrahydrofuran and treated dropwise with a solution of 34 g (143 mmol) of ethyl 4-methylamino-2-methylthio-pyrimidine-5-carboxylate in 300 mL of dry tetrahydrofuran and left to stand for 15 minutes. The mixture was cooled in ice and cautiously treated dropwise with 18 mL of water. Sodium hydroxide solution (36 mL, 2M) was added dropwise, followed by 48 mL of water. The resulting suspension was stirred for 17 hours at room temperature and then filtered. The filter residue was washed twice with 100 mL of ethyl acetate. The filtrate and washings were combined and evaporated under reduced pressure. The residue was suspended in 200 mL of dichloromethane/hexane (2:1) and the solid was filtered and dried to give 23.5 g (86%) of 4-methylamino-2-methylthiopyrimidine-5-methanol as a yellow solid.

### Step 1.3 Preparation of 4-methylamino-2-methylthiopyrimidine-5-carboxaldehyde

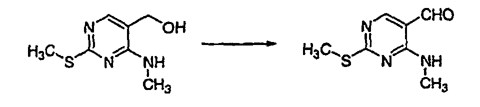

4-Methylamino-2-methylthiopyrimidine-5-methanol (20 g, 108 mmol) and 1 L of dichloromethane were combined with stirring and treated with 87 g (1 mol) of manganese dioxide. The resulting suspension was stirred for 24 hours and then filtered through a filter aid. The filter residue was washed with 100 mL of dichloromethane and the combined filtrate and washings were evaporated under reduced pressure to give 15.8 g (80%) of 4-methylamino-2-methylthiopyrimidine-5-carboxaldehyde as a white solid.

### Step 1.4

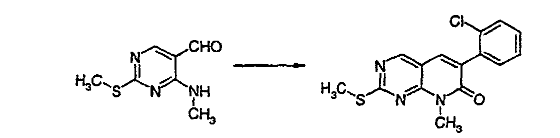

To a mixture of 3.3 g (18.1 mmol) of 4-methylamino-2-methylthiopyrimidine-5-carboxaldehyde, 4.0 g (20.1 mmol) of ethyl-2-chlorophenylacetate in 30 mL of NMP was added 1.5 g of resin, polymer bound 1,3,4,6,7,8-hexahydro-2H pyrimido[1,2-a]pyrimidine. The reaction mixture was heated to 120 °C. After 48 h, the reaction mixture was cooled to room temperature and filtered. The resin was washed with NMP and ethyl acetate. The filtrate was diluted with water and filtered. The product was isolated by filtration and by extraction of the filtrate with ethyl acetate. The product was washed with 5% aqueous HCl and water and dried to give 4.0 g of sulfide (mass spec. MH⁺ = 318. Mpt. 193.0-193.4).

### Step 1.5

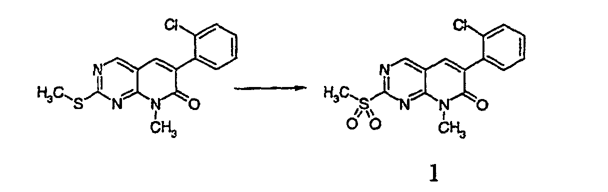

A solution of 13.5 g (42.5 mmol) of sulfide in chloroform was cooled in ice and treated with 20.5 g (91 mmol) of 3-chloroperbenzoic acid. The mixture was stirred at room temperature for 16 hours and diluted with saturated aqueous sodium bicarbonate solution. The phases were separated. The organic phase was dried over magnesium sulfate and filtered. The filtrate was concentrated under reduced pressure and the product was stirred in ethyl ether, filtered and dried to give 13.1 g of sulfone 1 (mass spec. MH⁺ = 350. Mpt. 232.6-232.8).

### Example 2

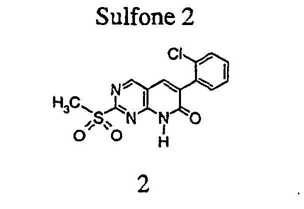

This example illustrates the preparation of 6-(2-chlorophenyl)-2-methanesulfonyl-pyrido[2,3-*d*]pyrimidin-7-ol starting with ethyl 4-chloro-2-methylthiopyrimidine-5-carboxylate.

### Step 2.1 Preparation of ethyl 4-amino-2-methylthiopyrimidine-5-carboxylate

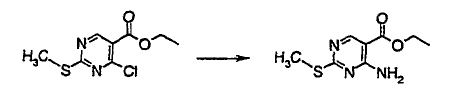

A solution of ethyl 4-chloro-2-methylthiopyrimidine-5-carboxylate (25.4 g, 106 mmol, Aldrich Chemical Co., Milwaukee, Wisconsin, USA) in 300 mL of tetrahydrofuran was treated with 50 mL of triethylamine and 40 mL of aqueous ammonium hydroxide. After stirring for 4 hours, 300 mL of water was added and the phases were separated. The organic layer was washed with 300 mL of brine, concentrated *in vacuo*, dissolved in methylene chloride, dried over sodium sulfate, filtered and concentrated *in vacuo* to give 16.5 g (95 %) of ethyl 4-amino-2-methylthiopyrimidine-5-carboxylate as a white solid.

### Step 2.2 Preparation of 4-amino-2-methylthiopyrimidine-5-methanol

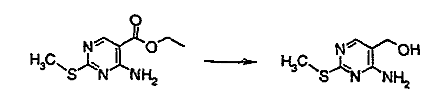

To a 0 °C solution of lithium aluminum hydride (175 mL, 175 mmol) in diethyl ether was added dropwise a solution of 4-amino-2-methylthiopyrimidine-5-carboxylate (34.7 g, 163 mmol) in 500 mL of dry tetrahydrofuran over a period of 1.5 hours. The reaction mixture was slowly warmed to ambient temperature and then cooled back to 0 °C before carefully quenching with 7 mL of water, 7 mL of 2 M sodium hydroxide solution, followed by 14 mL of water. The resulting suspension was filtered and the residue was washed with 2x300 mL of ethyl acetate. The filtrates were combined and concentrated to give 23.0 g (83 %) of 4-amino-2-methylthiopyrimidine-5-methanol as a white solid.

### Step 2.3 Preparation of 4-amino-2-methylthiopyrimidine-5-carboxaldehyde

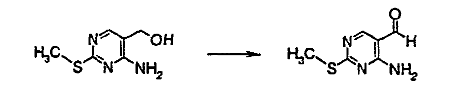

A suspension of 4-amino-2-methylthiopyrimidine-5-methanol (21.8 g, 128 mmol) in 800 mL of methylene chloride was treated with activated manganese oxide powder (63.0 g, 725 mmol). The reaction mixture was stirred for 18 hours, then filtered through a pad of celite. The solid residue was repeatedly washed with a solution of hot methylene chloride and methanol. The filtrates were combined and concentrated to give 17.5 g (81%) of 4-amino-2-methylthiopyrimidine-5-carboxaldehyde as a white solid.

### Step 2.4 Preparation of 6-(2-chlorophenyl)-2-methylthio-pyrido[2,3-d]pyrimidin-7-ol

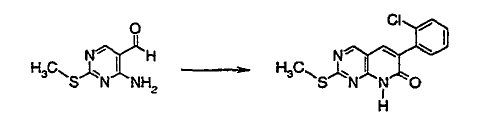

To a solution of 4-amino-2-methylthiopyrimidine-5-carboxaldehyde (21.7 g, 128 mmol) and ethyl-2-chlorophenylacetate (31.3 g, 158 mmol) in 250 mL of dry 1-methyl-2-pyrrolidinone was added potassium carbonate (63.0 g, 491 mmol). The reaction mixture was stirred at 95 °C for 16 hours and monitored by TLC (20:80, ethyl acetate/hexanes). An additional 12.0 g (60 mmol) of ethyl-2-chlorophenylacetate was added and the reaction mixture was stirred at 95 °C for another 16 hours. The reaction mixture was cooled and filtered. The filtered solids were washed with ethyl acetate. The filtrates were combined and diluted with 400 mL of water and 300 mL of ethyl acetate. The phasés were separated, and the organic layer was washed with brine, dried over sodium sulfate, filtered and concentrated *in vacuo* until a yellow precipitate formed. The solids were washed with ethyl acetate and dried to yield a minor amount of product. Most of the product remained in the aqueous layer and slowly precipitated out upon standing. The resulting suspension that formed was filtered and washed with water and ethyl acetate. This procedure was repeated six times yielding a total of 31.9 g (82%) of 6-(2-chlorophenyl)-2-methanesulfonyl-pyrido[2,3-d]pyrimidin-7-ol. Mass spec. M⁺ = 303, mp = 234.5-235.3 °C.

### Step 2.5 Preparation of 6-(2-chlorophenyl)-2-methanesulfonyl-pyrido[2,3-d]pyrimidin-7-ol

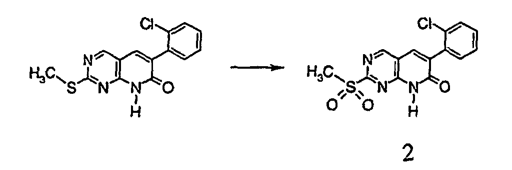

To a solution of 6-(2-chlorophenyl)-2-methylthio-pyrido[2,3-d]pyrimidin-7-ol (25.2 g, 82.9 mmol) was added a slurry of Oxone™ (105 g, 171 mmol) in 200 mL of water. The reaction mixture was stirred for 5 hours, filtered and concentrated *in vacuo*. The resulting slurry was filtered and the collected solids were successively washed with water four times and dried to give 23.2 g (83%) of 6-(2-chlorophenyl)-2-methanesulfonyl-pyrido[2,3-d]pyrimidin-7-ol as a light-yellow solid. Mass spec. MH⁺ = 336, mp = 215.1-221.1°C.

### Example 3

This example illustrates the preparation of 6-(2-chlorophenyl)-2-[(1-methyl-piperidin-4-ylmethyl)-amino]-8H-pyrido[2,3-d]pyrimidin-7-one.

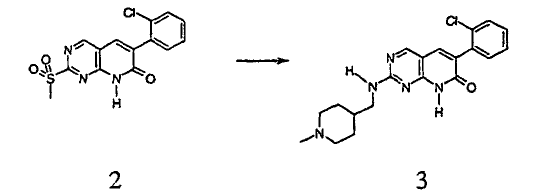

The sulfone 2 (0.5 g, 1.49 mmol) was combined with 1-methyl-4-aminomethylpiperidine (0.57 g, 4.47 mmol). The mixture was heated to 110 °C and stirred for 2 hours. The reaction was cooled to room temperature and the residue was dissolved in methanol/dichloromethane, and purified by column chromatography on silica gel in 10% methanol/dichloromethane. The fractions containing the product was combined and concentrated. The resulting residue was dissolved in 15 ml of 10% methanol/dichloromethane and treated with 1 equivalent of 1M HCl in ether. The solution was concentrated to a residue and triturated in ether. The solid was filtered and dried to yield 0.25 g of 6-(2-chlorophenyl)-2-[(1-methyl-piperidin-4-ylmethyl)-amino]-8H-pyrido[2,3-d]pyrimidin-7-one, HCl salt. Mass spec. MH⁺ = 383, melting pt. = 213 - 220 °C.

### Example 4

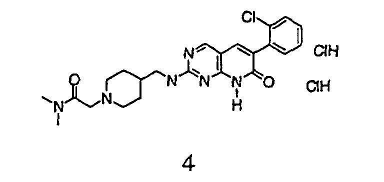

### Step 4.1 Preparation of 4B

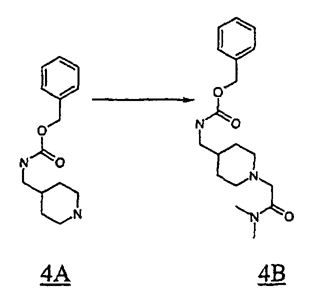

To a solution of compound 4A (4.704 g, 18.94 mmol) in DMF (30 mL) was added sodium carbonate (2.2 g, 1.1 eq) followed by 2-chloro-N,N-dimethyl acetamide (2.14 mL, 1.1 eq). The resulting mixture was stirred vigorously at room temperature for 18 hours. TLC indicated that more than 50% starting material remained so the reaction was heated to 80 °C for an additional 24 hours. Once again, TLC analysis showed that there was a substantial amount of starting material remaining, so the mixture was cooled to room temperature and then additional 2-chloro-N,N-dimethyl acetamide (0.58 mL, 0.3 eq) was added. The reaction was heated to 80 °C for another 4.5 hours whereby analysis by TLC indicated that there was very little starting material remaining. Then, ethyl acetate (150 mL) and water (50 mL) were added, and the mixture was partitioned and the layers were separated. The aqueous layer was further extracted with ethyl acetate (1X50 mL) and the combined organic layers were washed with brine (3X35 mL). The organic layers were combined, dried over magnesium sulfate, filtered and concentrated and dried under vacuum to give 9.9 g of crude product. Purification by flash chromatography using silica gel and gradient elution (neat dichloromethane to 10% methanol in dichloromethane) afforded compound 4B (3.91 g) as a thick syrup. MH⁺ = 334.

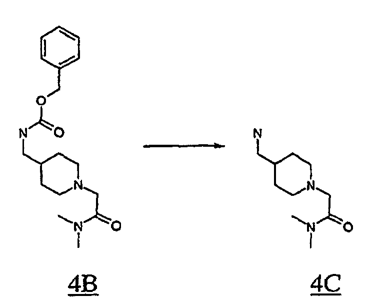

### Step 4.2 Preparation of 4C

The compound 4B was taken up in ethanol (120 mL) and nitrogen gas was gently bubbled over the solution for 5 minutes. Then 10% palladium on activated charcoal (1.5 g) was added and the mixture was placed under 1 atmosphere of hydrogen gas and stirred at room temperature for 18 hours. Analysis by TLC indicated that the reaction was complete so the mixture was filtered through a 2.5 cm bed of celite. The filtrate was concentrated and dried under vacuum to afford the compound 4C, 4-aminomethyl-1-dimethylaminocarbonylmethyl-piperidine (2.15 g) as an oil. (M+H)⁺= 200.

### Step 4.3 Preparation of 4

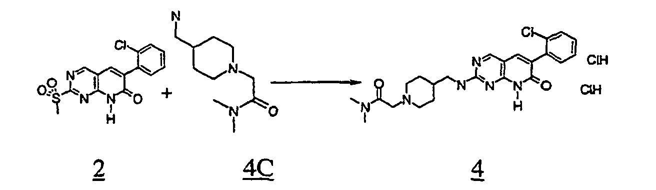

The sulfone 2 (200 mg, 0.614 mmol), the compound 4C (367 mg, 3 eq) and N-methyl pyrolidinone (0.3 mL) were mixed in a 10 mL flask and heated at 110 °C with stirring. After 5 minutes, the fluid mixture turned to a solid and by TLC analysis the reaction was complete. The reaction mixture was cooled and methanol (20 mL) was added. The precipitate was crushed up and then filtered to yield a white powder (235 mg). M.p.=263.1-263.5 °C, (M+H)⁺ =455. This free amine (230 mg) was dissolved in dichloromethane (50 mL) and methanol (50 mL) and HCl gas was bubbled through the solution for 15 minutes. The vessel was capped tightly and stirred for 2 hours. Then the solvent was removed under reduced pressure at 50 °C and coevaporated with dichloromethane two times. The resulting HCl salt was dried under vacuum at 56 °C for 8 hours to give the compound 4 (276 mg) as an off-white solid. M.p.=211.8-212.8 °C, (M+H)⁺=455 (free base).

### Example 5

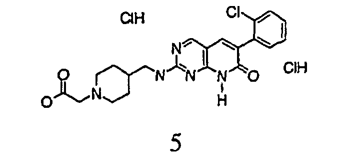

### Step 5.1 Preparation of 5A

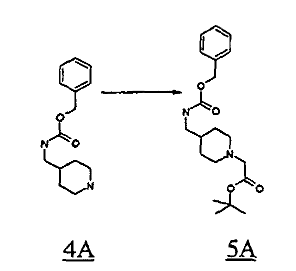

The compound 4A (4.632 g, 18.65 mmol) was dissolved in dimethylformamide (30 mL). To this solution was added sodium carbonate (2.17 g, 1.1 eq) and t-butyl bromoacetate (3 mL, 1.1 eq). The resulting mixture was stirred vigorously at room temperature for 18 hours. TLC analysis showed that very little starting material was present. Ethyl acetate (100 mL) and water (100 mL) were added to the reaction, and the mixture was partitioned and the layers were separated. The aqueous layer was further extracted with ethyl acetate (2X100 mL). The organic layers were combined, washed with water (1X75 mL) and brine (1X75 mL), dried over magnesium sulfate, filtered, concentrated and dried under vacuum to give 6.5 g of the crude product. Purification by flash column chromatography on silica gel using 3% methanol in dichloromethane as the eluent gave 5A (3.95 g)'as a thick syrup, (M+H)⁺ = 363.

### Step 5.2 Preparation of 5B

The compound 5A (3.95 g, 10.9 mmol) was dissolved in ethanol (125 mL) and nitrogen gas was gently passed over the mixture for 5 minutes, then 10% palladium on activated charcoal (1.5 g) was added. The resulting mixture was placed under 1 atmosphere of hydrogen gas and stirred for 18 hours. TLC analysis indicated that the reaction was complete and the reaction was filtered through a 2.5 cm bed of celite. The filtrate was concentrated and dried under vacuum to afford the comopund 5B, 4-aminomethyl-1-*tert*-butyloxycarbonylmethyl-piperidine as a colorless oil (2.26 g). (M+H)⁺ = 229.

### Step 5.3 Preparation of compound 5

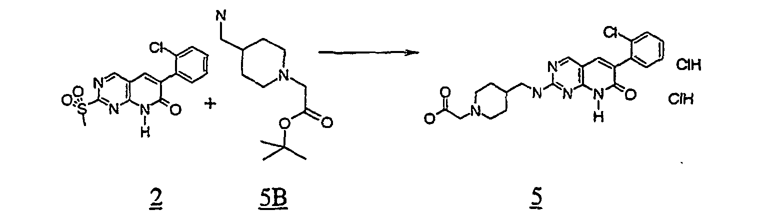

The sulfone 2 (200 mg, 0.614 mmol), compound 5B (420 mg, 3 eq), and N-methyl pyrrolidinone were mixed in a 10 mL flask and heated to 110 °C with stirring. The fluid mixture became semi-solid after about 10 minutes. The mixture was stirred for another 20 minutes and TLC analysis showed that the reaction was complete. Then 15 mL of ethyl acetate and 100 mL hexanes were added to the reaction mixture. The precipitated product was crushed up and filtered to provide a white powder. The powder was washed with 60 mL of hexanes and vacuum dried to yield 270 mg of the free amine t-butyl ester as a white powder. M.p.=217.6-220.0°C, (M+H)⁺ = 484. The free amine was dissolved in dioxane (100 mL) and HCl gas was bubbled through the solution for 15 minutes resulting in a homogeneous solution. The vessel was capped tightly and stirred at room temperature for 18 hours. The resulting precipitate was filtered and dried at 56 °C for 8 hours to give the HCl salt 11 (200 mg). M.p.=235.3-237.9 °C, (M+H)⁺ = 428 (free amine carboxylic acid).

### Example 6

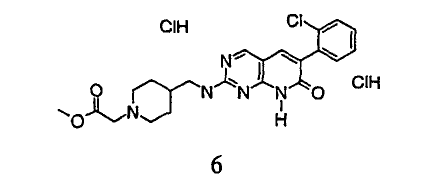

### Step 6.1 Preparation of 6A

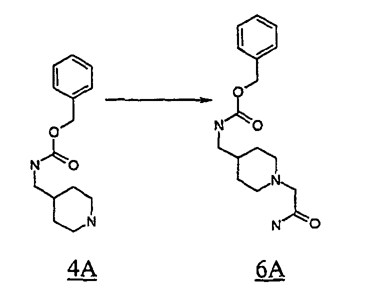

The compound 4A (4.889 g, 19.69 mmol) was dissolved in dimethylformamide (30 mL) and sodium carbonate (2.3 g, 1.1 eq) was added followed by 2-bromoacetamide (2.99 g, 1.1 eq) and the resulting mixture was stirred vigorously at room temperature for 18 hours. Analysis by TLC showed that the reaction was nearly complete. Ethyl acetate (150 mL) and water (50 mL) were added, and the mixture was partitioned and the layers were separated. The organic layer was washed with water (2X50 mL) and brine (1X75 mL), dried over magnesium sulfate, filtered, concentrated and vacuum dried to provide a solid. Hexanes (300 mL) were added to the residue and the solids were crushed up and mixed well. The supernatant was then decanted. This procedure was repeated again with 300 mL of hexanes. The residue was dried under vacuum to give a white powder 6A (3.13 g). (M+H)⁺ = 306.

### Step 6.2 Preparation of 6B

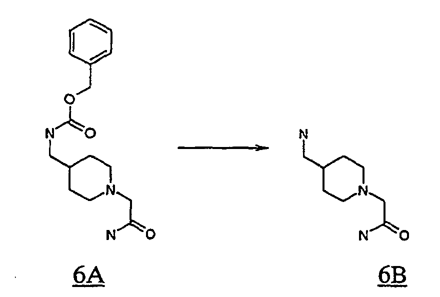

The compound 6A (3.1 g, 10.2 mmol) was dissolved in ethanol (250 mL) and nitrogen gas was gently bubbled through the mixture for 5 minutes. A mixture of 10% palladium on activated carbon (1.45 g) was added. The resulting mixture was placed under 1 atmosphere of hydrogen and stirred for 18 hours. The mixture was filtered through a 2.5 cm bed of celite. The filtrate was concentrated under reduced pressure at 40 °C and dried under vacuum to give the compound 6B (1.77 g) as a sticky white solid. (M+H)⁺ = 172.

### Step 6.3 Preparation of the compound 6

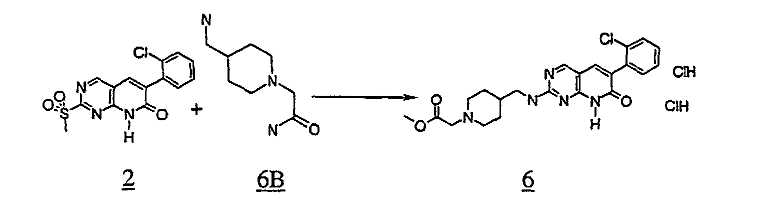

The sulfone 2 (400 mg, 1.23 mmol), the compound 6B (630 mg, 3 eq) and N-methyl pyrolidinone (0.3 mL) were mixed in a 25 mL flask and the mixture was heated to 110 °C with stirring for 30 minutes. The fluid mixture became a solid and TLC analysis indicated that the reaction was complete. The reaction mixture was diluted with about 20 mL of methanol and the white solid was filtered and dried to give 410 mg of the free amine primary amide. M.p.=244.6-245.9 °C, (M+H)⁺=427. This free amine primary amide was then dissolved in methanol (100 mL) and HCl gas was bubbled through the solution for 10 minutes. The vessel was then capped tightly and stirred at room temperature for 3 days. The solvent was removed under reduced pressure at 40 °C and then 10 mL of methanol was added to the residue. To the resulting solution was added tetrahydrofuran (100 mL) and the precipitate that was formed was filtered and collected to give the compound 6 as an off-white powder (250 mg). M.p.=220.0-221.1 °C, (M+H)⁺=442 (free amine, methyl ester).

### Example 7

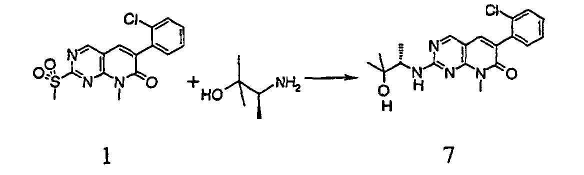

To a solution of the compound 9B (0.28 g, 2 mmol) in acetonitrile (5 mL) at room temperature was added TMSCN (0.8 mL, 3 eq). The resulting mixture was heated to 80 °C with stirring until the mixture was homogeneous. Then, sulfone 1 (0.35 g, 1 mmol) was added and the reaction was stirred at 80 °C for 40 minutes. The reaction was quenched with methanol (10 mL) and stirred for 5 minutes. After concentrating under reduced pressure at 50 °C, ethyl acetate (35 mL) and water (25 mL) were added to the residue. The organic layer was separated, washed with water (2X25 mL) and brine (1X25 mL), dried over magnesium sulfate, filtered, concentrated and dried to give 408 mg of crude material. Purification by preparative thin layer chromatography afforded the amine as an off-white powder (299 mg). (M+H)⁺=373, M.P.=91.4-93.2 °C. The free amine was dissolved in ethyl acetate (10 mL) and with stirring at room temperature was added a solution of 1M HCl in diethyl ether (1.2 mL, 1.5 eq). After stirring for 30 minutes, the solvent was removed under reduced pressure at 55 °C. Further concentration under high vacuum at 56 °C for 6 hours gave compound 7 as an off-white powder (275 mg). (M+H)⁺=373, M.P.=178.0-181.5 °C.

### Example 8

This example illustrates the preparation of 6-(2-chlorophenyl)-2-[(1,1-dimethyl-2-hydroxyethyl)amino]-8H-pyrido[2,3-d]pyrimidin-7-one.

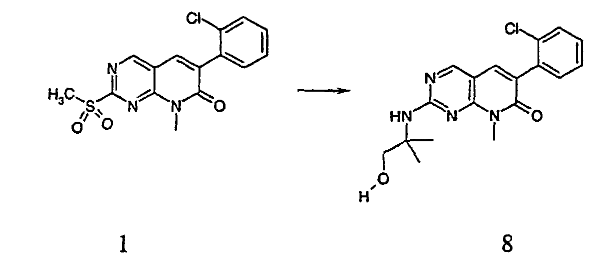

A mixture of 0.350 g (1.0 mmol) of sulfone 1 and 0.445 g (5.0 mmol) was stirred at 120 °C for 1 hour and then cooled to room temperature. The crude product was purified by column chromatography (5% methanol/dichloromethane) to give the desired product as a foam. The residue was suspended in methanol and addition of hydrochloric acid (1.0 M/Et₂O, 1 equivalent), stirred for 20 minutes and concentrated under reduced pressure. The residue was stirred in a mixture of MeOH/Et₂O for 1 hour, and the product was filtered to provide a white solid. Yield 190 mg. Mpt. 228.6-228.9 °C (HCl salt).

### Example 9

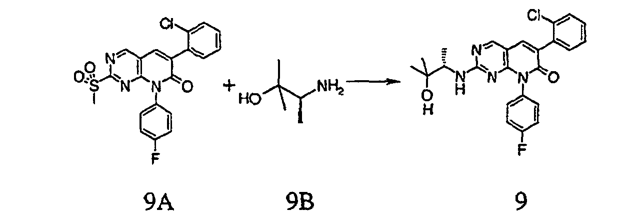

To a solution of compound 9B (Chem. Pharm. Bull. 45, 1997, 185-188) (0.28 g, 2 mmol) in acetonitrile (4 mL) at room temperature was added TMSCN (0.8 mL, 3 eq). The resulting mixture was heated to 80 °C until the mixture became homogeneous. Then 9A (0.4 g, 1 mmol) was added to the reaction mixture and stirred at 80 °C for 35 minutes. The reaction mixture was cooled to room temperature and 15mL of methanol was added and stirred for 5 minutes. The reaction mixture was concentrated under reduced pressure at 50 °C. The residue was diluted with ethyl acetate (35 mL) and water (25 mL). The organic phase was separated, washed with water (1X25 mL) and brine (1X25 mL), dried over magnesium sulfate, filtered and concentrated to yield 445 mg of crude material. Purification by preparative thin layer chromatography eluting with 50% ethyl acetate in hexanes gave the free amine as an off-white powder (242 mg). (M+H)⁺=453, M.P.=204.7-206.0 °C. The free amine was dissolved in ethyl acetate (15 mL) at room temperature and a solution of 1M HCl in diethyl ether (0.6 mL, 1.5 eq) was added. The resulting mixture was stirred for 2 hours. The solvent was removed under reduced pressure at 50 °C and the resulting solid was dried under vacuum at 56 °C to give compound 9 as an off-white powder (219 mg). (M+H)⁺=453, M.P.=142.0-149.0 °C.

### Example 10

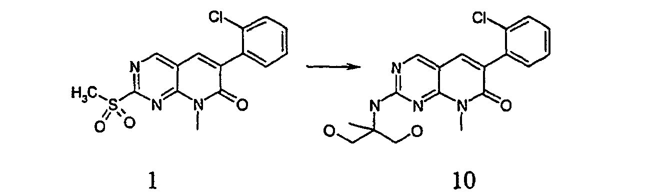

A mixture of sulfone 1 (250 mg, 0.71 mmol) and 2-amino-2-methyl-1,3-propanediol (150 mg, 1.4 mmol) in 1-methyl-2-pyrrolidinone (0.25 mL) was stirred at 80 °C for 12 h and then cooled to room temperature. Water (1 mL) was added and the suspension was stirred for 30 min, filtered and the precipitate was washed with water, dried and suspended in methanol. The suspension was again filtered and dried to give 83 mg of the desired product 10. Mass spec. MH⁺ = 374, mpt. 200-210.

### Example 11

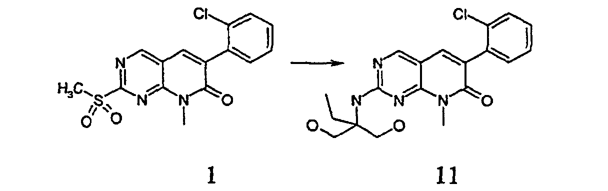

A mixture of sulfone 1 (250 mg, 0.71 mmol) and 2-amino-2-ethyl-1,3-propanediol (170 mg, 1.4 mmol) in 1-methyl-2-pyrrolidinone (0.25 mL) was stirred at 80 °C for 12 h and then cooled to room temperature. Water (1 mL) was added, and the suspension was stirred for 30 min, filtered and the precipitate was washed with water, dried and suspended in methanol. The suspension was again filtered and dried to give 83 mg of the desired product 11. Mass spec. MH⁺ = 388, mpt. 98.1-102.

### Example 12

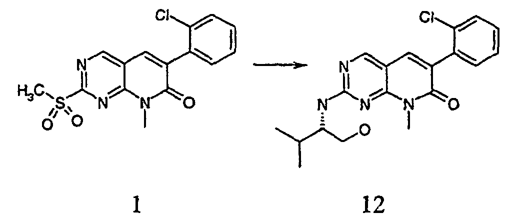

A mixture of sulfone 1 (250 mg, 0.71 mmol) and (S)-(+)-2-amino-3-methyl-1-butanol (147 mg, 1.4 mmol) in 1-methyl-2-pyrrolidinone (0.25 mL) was stirred at 80 °C for 12 h and then cooled to room temperature. Water (1 mL) was added, and the suspension was stirred for 30 min, filtered and the precipitate was washed with water, dried and suspended in methanol. The suspension was again filtered and dried to give 90 mg of the desired product 12. Mass spec. MH⁺ = 372, mpt. 167.1-169.1.

### Example 13

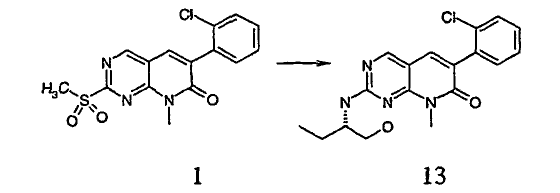

A mixture of sulfone 1 (250 mg, 0.71 mmol) and (S)-(+)-2-amino-1-butanol (127 mg, 1.4 mmol) in 1-methyl-2-pyrrolidinone (0.25 mL) was stirred at 80 °C for 12 h and then cooled to room temperature. Water (1 mL) was added, and the suspension was stirred for 30 min, filtered and the precipitate was washed with water, dried and suspended in methanol. The suspension was again filtered and dried to give 105 mg of the desired product 13. Mass spec. MH⁺ = 358, mpt. 170.5-172.1.

### Example 14

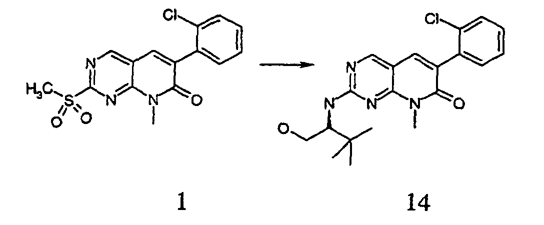

A mixture of sulfone 1 (250 mg, 0.71 mmol) and (S)-tert-leucinol (167 mg, 1.4 mmol) in 1-methyl-2-pyrrolidinone (0.25 mL) was stirred at 80 °C for 12 h and then cooled to room temperature. Water (1 mL) was added, and the suspension was stirred for 30 min, filtered and the precipitate was washed with water, dried and suspended in methanol. The suspension was again filtered and dried to give 116 mg of the desired product 14. Mass spec. MH⁺ = 386, mpt. 171.2-174.0.

### Example 15

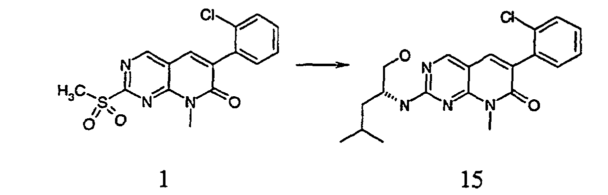

A mixture of sulfone 1 (250 mg, 0.71 mmol) and (R)-(-)-leucinol (167 mg, 1.4 mmol) in 1-methyl-2-pyrrolidinone (0.25 mL) was stirred at 80 °C for 12 h and then cooled to room temperature. Water (1 mL) was added, and the suspension was stirred for 30 min, filtered and the precipitate was washed with water, dried and suspended in methanol. The suspension was again filtered and dried to give 178 mg of the desired product 15. Mass spec. MH⁺ = 386, mpt.173.1-176.2.

### Example 16

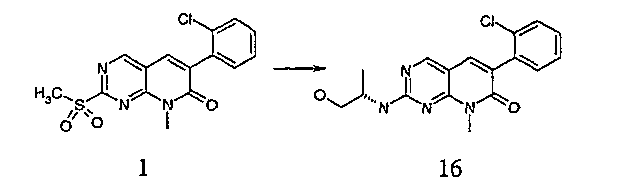

A mixture of sulfone 1 (250 mg, 0.71 mmol) and (S)-(+)-2-amino-1-propanol (107 mg, 1.4 mmol) in 1-methyl-2-pyrrolidinone (0.25 mL) was stirred at 80 °C for 12 h at and then cooled to room temperature. Water (1 mL) was added, and the suspension was stirred for 30 min, filtered and the precipitate was washed with water, dried and suspended in methanol. The suspension was again filtered and dried to give 87 mg of the desired product 16. Mass spec. MH⁺ = 344, mpt.131.1-132.2.

### Example 17

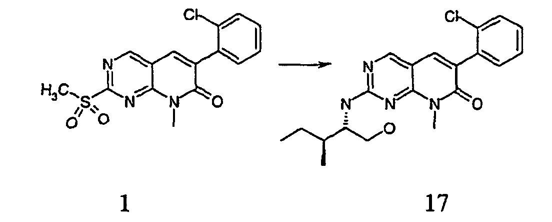

A mixture of sulfone 1 (250 mg, 0.71 mmol) and (S)-(+)-isoleucinol (167 mg, 1.4 mmol) in 1-methyl-2-pyrrolidinone (0.25 mL) was stirred at 80 °C for 12 h and then cooled to room temperature. Water (1 mL) was added, and the suspension was stirred for 30 min, filtered and the precipitate was washed with water, dried and suspended in methanol. The suspension was again filtered and dried to give 200 mg of the desired product 17. Mass spec. MH⁺ = 386, mpt.140.1-143.6.

### Example 18

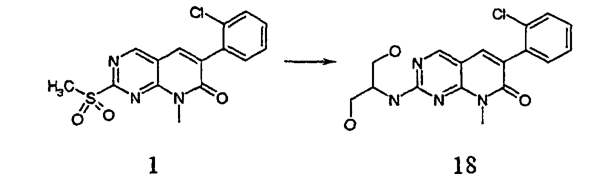

A mixture of sulfone 1 (250 mg, 0.71 mmol) and serinol (130 mg, 1.4 mmol) in 1-methyl-2-pyrrolidinone (0.25 mL) was stirred at 80 °C for 12 h and then cooled to room temperature. Water (1 mL) was added, and the suspension was stirred for 30 min, filtered and the precipitate was washed with water, dried and suspended in methanol. The suspension was again filtered and dried to give 179 mg of the desired product 18. Mass spec. MH⁺ = 360, mpt.155.8-157.3.

### Example 19

This example illustrates an alternative method for producing 6-(2-chlorophenyl)-8-methyl-2-methylthio-8-hydropyridino[2,3-d]pyrimidin-7-one (VI)

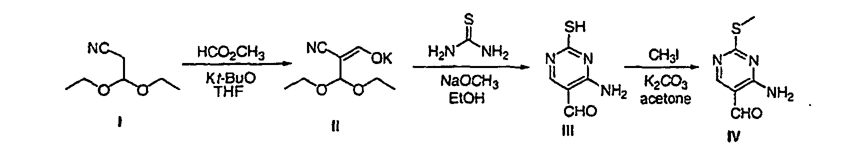

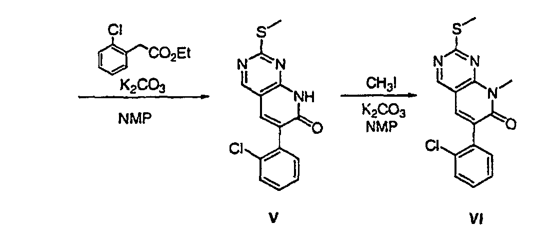

### Preparation of 3,3-Diethoxy-2-formylpropionitrile Potassium Salt (II)

To a stirred solution of 3,3-diethoxypropane-nitrile (I, 283.80 g, 1.98 moles) and methyl formate (148.80 g, 2.48 moles) in anhydrous THF (1.1 L) at 10°C was added 1.0 M potassium *tert*-butoxide in THF (2.2 L, 2.2 moles). Temperature was maintained in the range of 10 °C to 15 °C throughout the 45 minute addition. Following the addition, the resulting slurry was stirred 2 hours at ambient room temperature. Hexane (400 mL) was then added and stirring was continued for another 20 min. The slurry was filtered and the cake washed with 1/1 hexanes/THF and dried overnight at 60 °C in a vacuum oven. The yield of pale tan powder was 302.5 grams (73.0%). ¹H-NMR (CD₃OD) was consistent with the desired structure II.

### Preparation of 4-Amino-2-sulfanylpyrimidine-5-carbaldehyde (III)

A slurry of thiourea (92.8 g, 1.22 moles) in ethanol (90 mL) was heated under reflux and vigorously stirred. To this slurry was added a suspension of 3,3-diethoxy-2-formylpropionitrile potassium salt II (222.20 g, 1.06 moles) in 25% sodium methoxide/methanol (85.5 mL, 0.37 mole) and ethanol (285 mL) in five aliquots over a 10 minute period while maintaining reflux conditions (alternatively, the latter slurry may be heated to 50°C to give a homogenous solution for the addition). An additional portion of ethanol (150 mL) was added to facilitate stirring. The thick slurry became a bright yellow color following the addition and was held under reflux for an additional 1 hour. The mixture was then cooled and evaporated to near dryness on a rotoevaporator. The residue was dissolved in water (940 mL). Crude product was precipitated from solution by the addition of 30% acetic acid (280 mL) and isolated via filtration using a medium frit sintered glass filtration funnel. The cake was washed with water (800 mL). Purification via trituration in hot water (1 L) for 30 minutes, followed by cooling and filtration gave 118.9 grams (72.3%) of product as a bright yellow solid after drying overnight at 60 °C in a vacuum oven (subsequent preparations have demonstrated that this trituration is unnecessary). An HPLC gave purity as 98.67%. ¹H-NMR (DMSO-d₆) was consistent with desired structure III.

### Preparation of 4-Amino-2-methylthiopyrimidine-5-carbaldehyde (IV)

To a solution of 4-amino-2-sulfanyl-pyrimidine-5-carbaldehyde III (100.00 g, 644.4 mmoles) and 325 mesh potassium carbonate (178.10 g, 1.29 moles) in acetone (1.5 L) was added iodomethane (128.10 g, 902.2 mmoles) dropwise over 20 minutes with mild cooling. The mixture was stirred at ambient room temperature over the weekend. TLC showed remaining III and an additional aliquot of iodomethane was added (8 mL) and stirring was continued overnight. TLC again showed some III remaining and an addition portion of iodomethane was added (8 mL) and stirring was continued another 24 hour period. An HPLC showed 95.9% S-alkylated product and 3.7% of compound III. The reaction mixture was stripped to near dryness on a rotoevaporator. Water (1 L) was added to the residue and the product was collected via filtration and washed with water (200 mL). The product was dried overnight in a vacuum oven at 60 °C. Yield was 103.37 grams (94.8%). An HPLC showed 95.8% IV and 4.2% III.

### Preparation of 6-(2-chlorophenyl)-2-methylthio-8-hydropyridino[2,3-d]pyrimidin-7-one (V)

A mixture of IV (10.00 g, 59.1 mmoles), ethyl 2-(2-chlorophenyl)acetate (14.40 g, 71.8 mmoles), NMP (115 mL) and 325 mesh potassium carbonate (29.00 g, 209.8 mmoles) was heated at 95 °C overnight. The reaction mixture was cooled and diluted with water (800 mL). The resulting slurry was stirred overnight and filtered to isolate product (V). The filter cake was washed with water and dried at 60 °C in a vacuum oven overnight. Isolated yield was 14.9 grams (83.0%) of dark tan solid.
Analysis by an HPLC showed 98.3% purity.

### Preparation of 6-(2-Chlorophenyl)-8-methyl-2-methylthio-8-hydropyridino[2,3-d]pyrimidin-7-one (VI)

A mixture of V (0.25 g, 0.82 mmole), NMP (5 mL), potassium carbonate (0.11 g, 0.82 mmole), and iodomethane (0.14 g, 0.96 mmole) was stirred under nitrogen at ambient room temperature overnight. Water (15 mL) was added and stirring was continued for 24 hours. The slurry was filtered and the filter cake washed with water (10 mL). An HPLC showed 97.8% purity.

### Example 20

This example illustrate an assay protocol for determining *in vitro* inhibition of p-38 (MAP) Kinase.

The p-38 MAP kinase inhibitory activity of compounds of this invention *in vitro* was determined by measuring the transfer of the γ-phosphate from γ-³³P-ATP by p-38 kinase to Myelin Basic Protein (MBP), using the a minor modification of the method described in Ahn, N. G.; et al. *J. Biol. Chem*. Vol. 266(7), 4220-4227, (1991).

The phosphorylated form of the recombinant p38 MAP kinase was expressed with SEK-1 and MEKK in E. Coli and then purified by affinity chromatography using a Nickel column.

The phosphorylated p38 MAP kinase was diluted in kinase buffer (20 mM 3-(N-morpholino)propanesulfonic acid, pH 7.2, 25 mM β-glycerol phosphate, 5 mM ethylene glycol-bis(beta-aminoethyl ether)-N,N,N',N'-tetraacetic acid, 1mM sodium vanadate, 1mM dithiothreitol, 40mM magnesium chloride). Test compound dissolved in DMSO or only DMSO (control) was added and the samples were incubated for 10 min at 30 °C. The kinase reaction was initiated by the addition of a substrate cocktail containing MBP and γ-³³P-ATP. After incubating for an additional 20 min at 30 °C, the reaction was terminated by adding 0.75% phosphoric acid. The phosphorylated MBP was then separated from the residual γ-³³P-ATP using a phosphocellulose membrane (Millipore, Bedford, MA) and quantitated using a scintillation counter (Packard, Meriden, CT).

Compounds of the invention were active in this assay. The p-38 inhibitory activities (expressed as IC₅₀, the concentration causing 50 % inhibition of the p-38 enzyme being assayed) of some compounds of the invention are:

| CPD # (from Table 1) | IC₅₀, µM |
|---|---|
| 1 | 0.052 |
| 3 | 2.196 |
| 4 | 6.266 |
| 7 | 0.0003 |
| 8 | 0.031 |
| 10 | 0.042 |
| 11 | 0.024 |
| 12 | 0.124 |
| 13 | 0.048 |
| 18 | 0.092 |

### Example 21

This example illustrates an *in vitro* assay to evaluate the inhibition of LPS-induced TNF-α production in THP1 cells.

The ability of the compounds of this invention to inhibit the TNF-α release was determined using a minor modification of the methods described in Blifeld, *et al*. *Transplantation*, 51:498-503 (1991).

### (a) Induction of TNF biosynthesis:

THP-1 cells were suspended in culture medium [RPMI (Gibco-BRL, Gailthersburg, MD) containing 15% fetal bovine serum, 0.02 mM 2-mercaptoethanol], at a concentration of 2.5 x 10⁶ cells/mL and then plated in 96 well plate (0.2 mL aliquots in each well). Test compounds were dissolved in DMSO and then diluted with the culture medium such that the final DMSO concentration was 5%. Twenty five µL aliquots of test solution or only medium with DMSO (control) were added to each well. The cells were incubated for 30 min., at 37 °C. LPS (Sigma, St. Louis, MO) was added to the wells at a final concentration of 0.5 µg/ml, and cells were incubated for an additional 2 h. At the end of the incubation period, culture supernatants were collected and the amount of TNF-α present was determined using an ELISA assay as described below.

### (b) ELISA Assay:

The amount of human TNF-α present was determined by a specific trapping ELISA assay using two anti-TNF-α antibodies (2TNF-H12 and 2TNF-H34) described in Reimund, J. M., et al. *GUT.* Vol. 39(5), 684-689 (1996).

Polystyrene 96-well plates were coated with 50 µl per well of antibody 2TNF-H12 in PBS (10 µg/mL) and incubated in a humidified chamber at 4 °C overnight. The plates were washed with PBS and then blocked with 5% nonfat-dry milk in PBS for 1 hour at room temperature and washed with 0.1% BSA (bovine serum albumin) in PBS.

TNF standards were prepared from a stock solution of human recombinant TNF-α (R&D Systems, Minneapolis, MN). The concentration of the standards in the assay began at 10 ng/mL followed by 6 half log serial dilutions.

Twenty five µL aliquots of the above culture supernatants or TNF standards or only medium (control) were mixed with 25 µL aliquots of biotinylated monoclonal antibody 2TNF-H34 (2 µg/mL in PBS containing 0.1% BSA) and then added to each well. The samples were incubated for 2 hr at room temperature with gentle shaking and then washed 3 times with 0.1% BSA in PBS. 50 µl of peroxidase-streptavidin (Zymed, S. San Francisco, CA) solution containing 0.416 µg/mL of peroxidase-streptavidin and 0.1% BSA in PBS was added to each well. The samples were incubated for an additional 1 hr at room temperature and then washed 4 times with 0.1% BSA in PBS. Fifty µL of O-phenylenediamine solution (1 µg/mL O-phenylenediamine and 0.03 % hydrogen peroxide in 0.2M citrate buffer pH 4.5) was added to each well and the samples were incubated in the dark for 30 min., at room temperature. Optical density of the sample and the reference were read at 450 nm and 650 nm, respectively. TNF-α levels were determined from a graph relating the optical density at 450 nm to the concentration used. '

The IC₅₀ value was defined as the concentration of the test compound corresponding to half-maximal reduction in 450 nm absorbance.

### Example 22

This example illustrates an *in vivo* assay to evaluate the inhibition of LPS-induced TNF-α production in mice (or rats).

The ability of the compounds of this invention to inhibit the TNF-α release, *in vivo,* was determined using a minor modification of the methods described in in Zanetti, *et. al., J. Immunol*., 148:1890 (1992) and Sekut, *et. al., J. Lab. Clin. Med.,* 124:813 (1994).

Female BALB/c mice weighing 18-21 grams (Charles River, Hollister, CA, USA) were acclimated for one week. Groups containing 8 mice each were dosed orally either with the test compounds suspended or dissolved in an aqueous vehicle containing 0.9% sodium chloride, 0.5% sodium carboxymethyl-cellulose, 0.4% polysorbate 80, 0.9% benzyl alcohol (CMC vehicle) or only vehicle (control group). After 30 min., the mice were injected intraperitoneally with 20 µg of LPS (Sigma, St. Louis, MO, USA). After 1.5 h, the mice were sacrificed by CO₂ inhalation and blood was harvested by cardiocentesis. Blood was clarified by centrifugation at 15,600 X g for 5 min., and sera were transferred to clean tubes and frozen at -20°C until analyzed for TNF-α by ELISA assay (Biosource International, Camarillo, CA, USA) following the manufacturer's protocol.

## Claims

1. A compound of the formula: 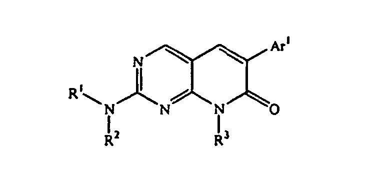 wherein:
R¹ is hydrogen or alkyl;
R² is -CR'R"-R^{a} (where R' and R" are hydrogen, hydroxyalkyl or alkyl with at least one being alkyl or hydroxyalkyl and R^{a} is hydroxyalkyl), wherein each of the hydroxy group present in R² can be independently in the form of an ester, a carbamate, a carbonate, or a sulfonate derivative;
R³ is hydrogen, alkyl, amino, monoalkylamino, dialkylamino, cycloalkyl, aryl, aralkyl, haloalkyl, heteroalkyl, cyanoalkyl, alkylene-C(O)-R (where R is hydrogen, alkyl, hydroxy, alkoxy, amino, monoalkylamino or dialkylamino) or acyl;
Ar¹ is aryl;
"aryl" means a monovalent monocyclic or bicyclic aromatic hydrocarbon radical which is optionally substituted independently with one, two or three, substituents selected from the group consisting of alkyl, hydroxy, alkoxy, haloalkyl, haloalkoxy, heteroalkyl, halo, nitro, cyano, amino, monoalkylamino, dialkylamino, methylenedioxy, ethylenedioxy and acyl;
"acyl" means a radical -C(O)R, where R is hydrogen, alkyl, cycloalkyl, cycloalkylalkyl, phenyl or phenylalkyl;
"heteroalkyl" means an alkyl radical as defined herein wherein one, two or three hydrogen atoms have been replaced with a substituent independently selected from the group consisting of -OR^{a}, -NR^{b}R^{c}, and -S(O)ₙR^{d} (where n is an integer from 0 to 2), with the understanding that the point of attachment of the heteroalkyl radical is through a carbon atom, wherein R^{a} is hydrogen, acyl, alkyl, cycloalkyl, or cycloalkylalkyl; R^{b} and R^{c} are independently of each other hydrogen, acyl, alkyl, cycloalkyl, or cycloalkylalkyl, or R^{b} and R^{c} together forms cycloalkyl or arylcycloalkyl; and when n is 0, R^{d} is hydrogen, alkyl, cycloalkyl, or cycloalkylalkyl, and when n is 1 or 2, R^{d} is alkyl, cycloalkyl, cycloalkylalkyl, amino, acylamino, monoalkylamino, or dialkylamino;
"cycloalkyl" refers to a saturated monovalent cyclic hydrocarbon radical of three to seven ring carbons; and
"alkyl" means a linear saturated monovalent hydrocarbon radical of one to six carbon atoms or a branched saturated monovalent hydrocarbon radical of three to six carbon atoms,
or a pharmaceutically acceptable salt thereof.

2. The compound of Claim 1 wherein Ar¹ is an optionally substituted phenyl.

3. The compound of Claim 2, wherein Ar¹ is a phenyl group independently substituted with one or two halo, alkyl or methoxy groups.

4. The compound of Claim 3, wherein Ar¹ is 2-chlorophenyl, 2-methylphenyl or 2-methoxyphenyl.

5. The compound according to claim 4 of the formula:

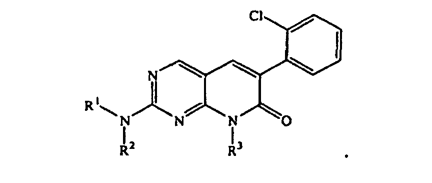

6. The compound according to any one of claims 2-5, wherein R¹ is hydrogen or methyl.

7. The compound according to claim 6, wherein R² is (1,1-dimethyl-2-hydroxy)ethyl or 1,2-dimethyl-2-hydroxy)propyl, wherein each of the hydroxy group present in R² can be independently in the form of an ester, a carbamate, a carbonate, or a sulfonate derivative.

8. A pharamceutical composition comprising a pharmaceutically acceptable excipient and a compound as claimed in any one of claims 1 to 7.

9. A process for preparing a compound of Claim 1, comprising the steps of contacting a compound of the formula Ig: 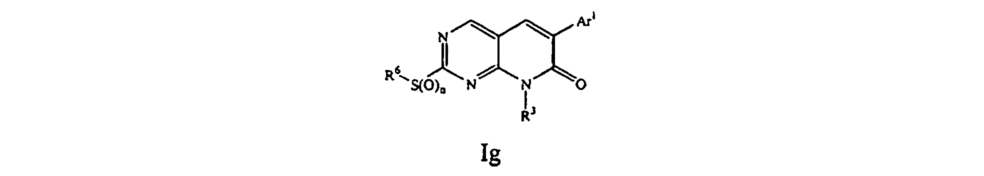 with an amine of the formula R¹R²NH under conditions sufficient to produce a compound of Formula I: 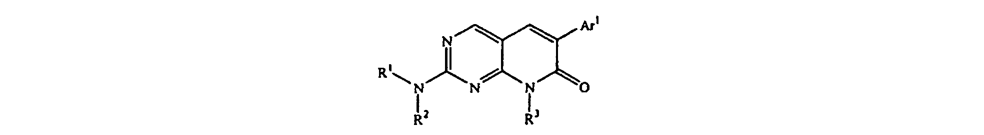 wherein:
R¹, R², R³ and Ar¹ are as defined in claim 1;
n is an integer from 0 to 2; and
R⁶ is an alkyl group.

10. The process of claim 9, wherein n is 1.

11. The process of claim 9 wherein n is 2.

12. A process for producing a pyrimidine of the formula: 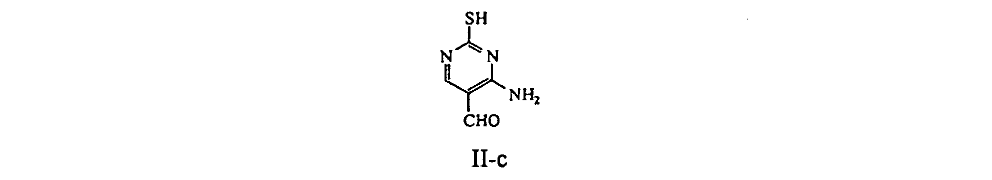 comprising:
(a) contacting an acetal of the formula NC-CH₂-CH(OR^{a})₂, with an alkyl formate of the formula HCO₂R in the presence of a condensation base under conditions sufficient to produce a condensed product; and
(b) contacting said condensed product with thiourea in the presence of a cyclization base under conditions sufficient to produce said pyrimidine of Formula II-c,
wherein each of R and R^{a} is independently alkyl.

13. The process of Claim 12, wherein said condensation base is a tert-butoxide.

14. The process of Claim 12, wherein said cyclization base is an alkoxide.

15. A process for producing a pyridopyrimidine of the formula: 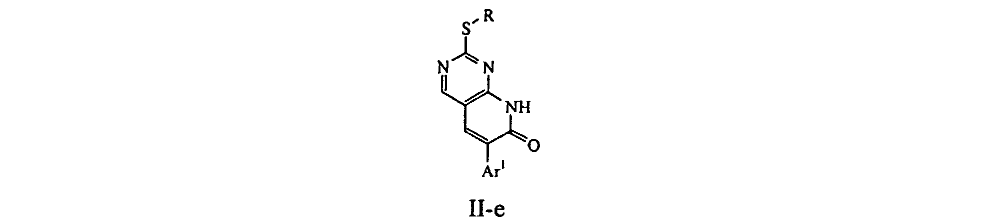 comprising:
(a) contacting a pyrimidine of the formula: 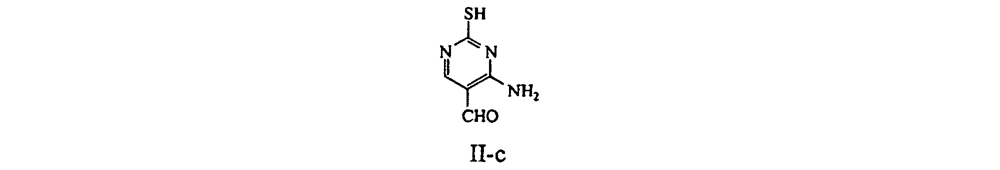 with an alkylating agent of the formula R-X¹ in the presence of an alkylating base under conditions sufficient to produce an alkylated pyrimidine of the formula: 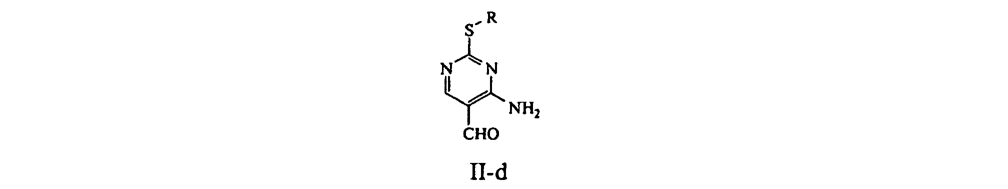 and
(b) contacting said alkylated pyrimidine of Formula II-d with an aryl acetate of the formula Ar¹-CH₂-CO₂R in the presence of a cyclization base under conditions sufficient to produce said pyridopyrimidine of Formula II-e;or
or
(a) contacting said pyrimidine of Formula II-c with said aryl acetate of the formula Ar¹-CH₂-CO₂R in the presence of a cyclization base under conditions sufficient to produce a thiol pyridopyrimidine of the formula: 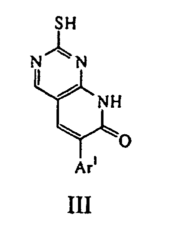 and
(b) contacting said thiol pyridopyrimidine of Formula III with said alkylating agent of the formula R-X¹ in the presence of an alkylating base under conditions sufficient to produce said pyridopyrimidine of Formula II,
wherein
X¹ is a leaving group;
each R is independently alkyl;
Ar¹ is aryl;

16. The process of Claim 15, wherein X¹ is halide.

17. The process of Claim 15 further comprising contacting said pyridopyrimidine of Formula II with a nitrogen alkylating agent of the formula R³-X² under conditions sufficient to produce an N-substituted pyridopyrimidine of the formula: 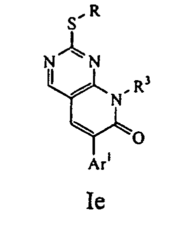 wherein
R³ is alkyl, amino, monoalkylamino, dialkylamino, cycloalkyl, aralkyl, haloalkyl, heteroalkyl, cyanoalkyl, alkylene-C(O)-R' (where R' is hydrogen, alkyl, hydroxy, alkoxy, amino, monoalkylamino or dialkylamino) or acyl;
X² is a leaving group; and
Ar¹ and R are those defined in Claim 15.

18. The process of Claim 17, wherein X² is halide.

19. Compounds as claimed in anyone of claims 1 to 7 as therapeutically active agents.

20. Use of one or more compounds as claimed in any of claims 1 to 7 for the preparation of a medicament for the prevention or treatment of arthritis, Crohns disease, Alzheimer's disease, irritable bowel syndrome, adult respiratory distress syndrome or chronic obstructive pulmonary disease.

21. The compound according to claim 1 having the formula 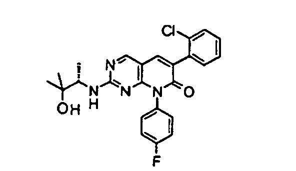 and pharmaceutically acceptable salts thereof.

22. The compound according to claim 1 having the formula 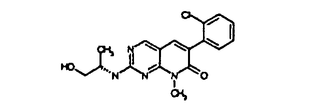 and pharmaceutically acceptable salts thereof.

23. The compound according to claim 1 having the formula 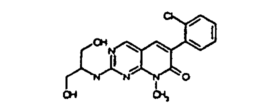 and pharmaceutically acceptable salts thereof.

## Patentansprüche

1. Verbindung der Formel: 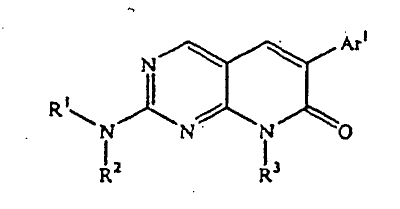 wobei:
R¹ Wasserstoff oder Alkyl ist;
R² -CR'R"-R^{a} ist (wobei R' und R" Wasserstoff, Hydroxyalkyl oder Alkyl sind, wobei mindestens einer davon Alkyl oder Hydroxyalkyl ist, und R^{a} Hydroxyalkyl ist), wobei die Hydroxygruppen in R² jeweils unabhängig in Form eines Ester-, eines Carbamat-, eines Carbonat- oder eines Sulfonatderivats vorliegen können;
R³ Wasserstoff, Alkyl, Amino, Monoalkylamino, Dialkylamino, Cycloalkyl, Aryl, Aralkyl, Halogenalkyl, Heteroalkyl, Cyanoalkyl, Alkylen-C(O)-R (wobei R Wasserstoff, Alkyl, Hydroxy, Alkoxy, Amino, Monoalkylamino oder Dialkylamino ist) oder Acyl ist; Ar¹ Aryl ist;
"Aryl" einen monovalenten monocyclischen oder bicyclischen aromatischen Kohlenwasserstoffrest bedeutet, welcher gegebenenfalls unabhängig mit einem, zwei oder drei Substituenten substituiert ist, ausgewählt aus Alkyl, Hydroxy, Alkoxy, Halogenalkyl, Halogenalkoxy, Heteroalkyl, Halogen, Nitro, Cyano, Amino, Monoalkylamino, Dialkylamino, Methylendioxy, Ethylendioxy und Acyl; "Acyl" einen Rest -C(O)R bedeutet, wobei R Wasserstoff, Alkyl, Cycloalkyl, Cycloalkylalkyl, Phenyl oder Phenylalkyl ist;
"Heteroalkyl" einen Alkylrest wie hier definiert bedeutet, wobei eines, zwei oder drei Wasserstoffatome durch einen Substituenten ersetzt worden sind, unabhängig ausgewählt aus -OR^{a}, -NR^{b}R^{c} und -S(O)ₙR^{d} (wobei n eine ganze Zahl von 0 bis 2 ist), mit der Maßgabe, dass die Bindung des Heteroalkylrests durch ein Kohlenstoffatom erfolgt, wobei R^{a} Wasserstoff, Acyl, Alkyl, Cycloalkyl oder Cycloalkylalkyl ist; R^{b} und R^{c} unabhängig voneinander Wasserstoff, Acyl, Alkyl, Cycloalkyl oder Cycloalkylalkyl sind oder R^{b} und R^{c} zusammen Cycloalkyl oder Arylcycloalkyl bilden; und wenn n 0 ist, R^{d} Wasserstoff, Alkyl, Cycloalkyl oder Cycloalkylalkyl ist, und wenn n 1 oder 2 ist, R^{d} Alkyl, Cycloalkyl, Cycloalkylalkyl, Amino, Acylamino, Monoalkylamino oder Dialkylamino ist;
"Cycloalkyl" einen gesättigten einwertigen cyclischen Kohlenwasserstoffrest mit drei bis sieben Ringkohlenstoffatomen betrifft; und
"Alkyl" einen linearen gesättigten einwertigen Kohlenwasserstoffrest mit ein bis sechs Kohlenstoffatomen oder einen verzweigten gesättigten einwertigen Kohlenwasserstoffrest mit drei bis sechs Kohlenstoffatomen bedeutet,
oder ein pharmazeutisch verträgliches Salz davon.

2. Verbindung nach Anspruch 1, wobei Ar¹ ein gegebenenfalls substituiertes Phenyl ist.

3. Verbindung nach Anspruch 2, wobei Ar¹ ein Phenylrest ist, welcher unabhängig mit einem oder zwei Halogenatomen, Alkylresten oder Methoxygruppen substituiert ist.

4. Verbindung nach Anspruch 3, wobei Ar¹ 2-Chlorphenyl, 2-Methylphenyl oder 2-Methoxyphenyl ist.

5. Verbindung nach Anspruch 4 der Formel:

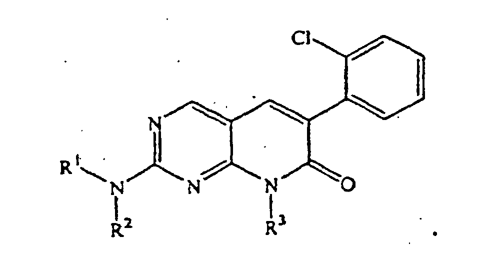

6. Verbindung nach einem der Ansprüche 2 bis 5, wobei R¹ Wasserstoff oder Methyl ist.

7. Verbindung nach Anspruch 6, wobei R² (1,1-Dimethyl-2-hydroxy)ethyl oder (1,2-Dimethyl-2-hydroxy)propyl ist, wobei die Hydroxygruppen in R² jeweils unabhängig in Form eines Ester-, eines Carbamat-, eines Carbonat- oder eines Sulfonatderivats vorliegen können.

8. Arzneimittel, umfassend einen pharmazeutisch verträglichen Exzipienten und eine Verbindung nach einem der Ansprüche 1 bis 7.

9. Verfahren zur Herstellung einer Verbindung nach Anspruch 1, umfassend die Schritte des Kontaktierens einer Verbindung der Formel Ig: 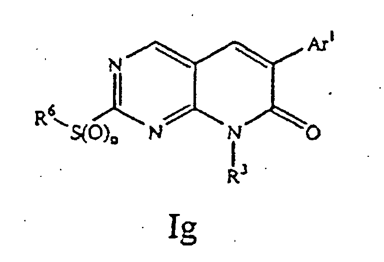 mit einem Amin der Formel R¹R²NH unter Bedingungen, die ausreichen, um eine Verbindung der Formel I: 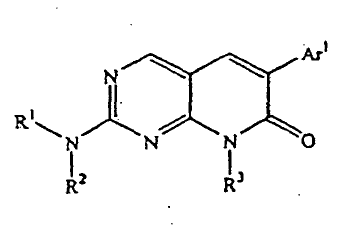 herzustellen,
wobei:
R¹, R², R³ und Ar¹ wie in Anspruch 1 definiert sind;
n eine ganze Zahl von 0 bis 2 ist; und
R⁶ ein Alkylrest ist.

10. Verfahren nach Anspruch 9, wobei n 1 ist.

11. Verfahren nach Anspruch 9, wobei n 2 ist.

12. Verfahren zur Herstellung eines Pyrimidins der Formel: 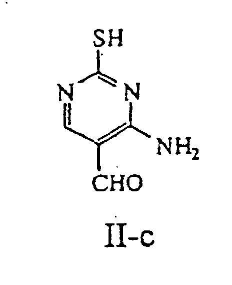 umfassend:
(a) Kontaktieren eines Acetals der Formel NC-CH₂-CH(OR^{a})₂ mit einem Alkylformiat der Formel HCO₂R in Gegenwart einer Kondensationsbase unter Bedingungen, die ausreichen, um ein kondensiertes Produkt herzustellen; und
(b) Kontaktieren des kondensierten Produkts mit Thioharnstoff in Gegenwart einer Cyclisationsbase unter Bedingungen, die ausreichen, um das Pyrimidin der Formel II-c herzustellen, wobei R und R^{a} jeweils unabhängig Alkyl sind.

13. Verfahren nach Anspruch 12, wobei die Kondensationsbase ein tert.-Butoxid ist.

14. Verfahren nach Anspruch 12, wobei die Cyclisationsbase ein Alkoxid ist.

15. Verfahren zur Herstellung eines Pyridopyrimidins der Formel: 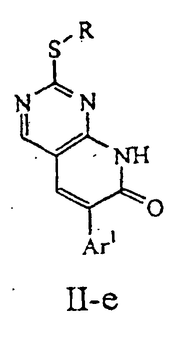 umfassend:
(a) Kontaktieren eines Pyrimidins der Formel: 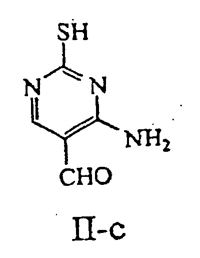 mit einem Alkylierungsmittel der Formel R-X¹ in Gegenwart einer Alkylierungsbase unter Bedingungen, die ausreichen, um ein alkyliertes Pyrimidin der Formel: 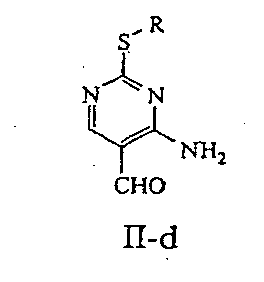 herzustellen,
und
(b) Kontaktieren des alkylierten Pyrimidins der Formel II-d mit einem Arylacetat der Formel Ar¹-CH₂-CO₂R in Gegenwart einer Cyclisationsbase unter Bedingungen, die ausreichen, um das Pyridopyrimidin der Formel II-e herzustellen; oder
(a) Kontaktieren des Pyrimidins der Formel II-c mit dem Arylacetat der Formel Ar¹-CH₂-CO₂R in Gegenwart einer Cyclisationsbase unter Bedingungen, die ausreichen, um ein Thiolpyridopyrimidin der Formel: 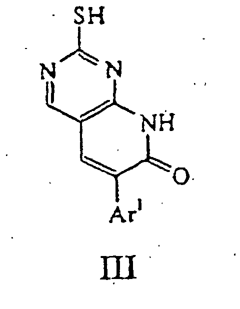 herzustellen,
und
(b) Kontaktieren des Thiolpyridopyrimidins der Formel III dem Alkylierungsmittel der Formel R-X¹ in Gegenwart einer Alkylierungsbase, unter Bedingungen, die ausreichen, um das Pyridopyrimidin der Formel II herzustellen,
wobei
X¹ eine Abgangsgruppe ist;
R jeweils unabhängig Alkyl ist;
Ar¹ Aryl ist.

16. Verfahren nach Anspruch 15, wobei X¹ ein Halogenid ist.

17. Verfahren nach Anspruch 15, ferner umfassend Kontatieren des Pyridopyrimidins der Formel II mit einem Stickstoff-Alkylierungsmittel der Formel R³-X² unter Bedingungen, die ausreichen, um ein N-substitutiertes Pyridopyrimidin der Formel: 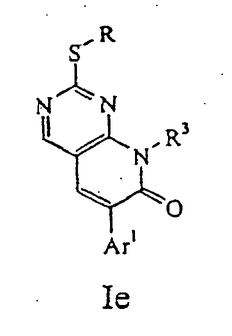 herzustellen,
wobei
R³ Alkyl, Amino, Monoalkylamino, Dialkylamino, Cycloalkyl, Aralkyl, Halogenalkyl, Heteroalkyl, Cyanoalkyl, Alkylen-C(O)-R' (wobei R' Wasserstoff, Alkyl, Hydroxy, Alkoxy, Amino, Monoalkylamino oder Dialkylamino ist) oder Acyl ist;
X² eine Abgangsgruppe ist; und
Ar¹ und R wie in Anspruch 15 definiert sind.

18. Verfahren nach Anspruch 17, wobei X² ein Halogenid ist.

19. Verbindungen nach einem der Ansprüche 1 bis 7 als therapeutische Wirkstoffe.

20. Verwendung einer oder mehrerer Verbindungen nach einem der Ansprüche 1 bis 7 zur Herstellung eines Medikaments zur Vorbeugung oder Behandlung von Arthritis, Morbus Crohn, Alzheimer, Reizkolon, ARDS oder chronisch-obstruktiver Lungenerkrankung.

21. Verbindung nach Anspruch 1 mit der Formel 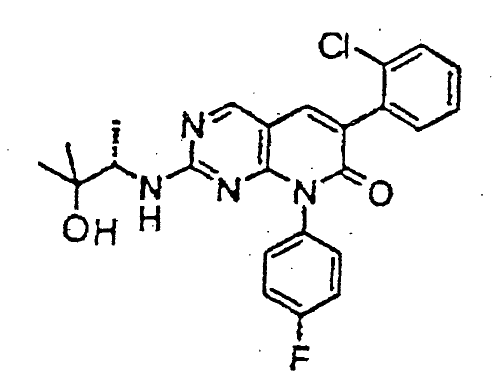 und pharmazeutisch verträgliche Salze davon.

22. Verbindung nach Anspruch 1 mit der Formel 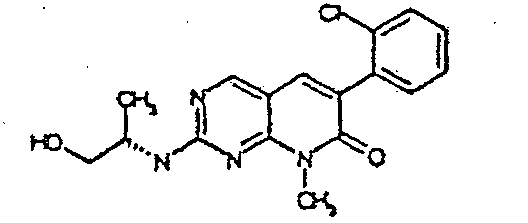 und pharmazeutisch verträgliche Salze davon.

23. Verbindung nach Anspruch 1 mit der Formel 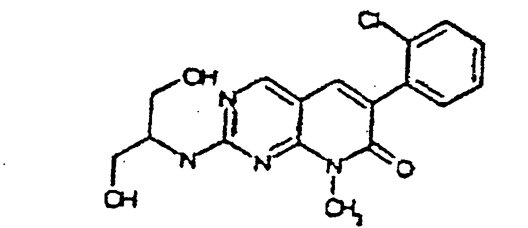 und pharmazeutisch verträgliche Salze davon.

## Revendications

1. Composé de formule : 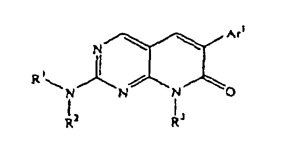 dans laquelle
R¹ est un hydrogène ou un alkyle ;
R² est -CR'R"-R^{a} (où R' et R" sont un hydrogène, un hydroxyalkyle ou un alkyle, au moins un d'entre eux étant un alkyle ou un hydroxyalkyle, et R^{a} est un hydroxyalkyle), où chaque groupe hydroxy présent dans R² peut être, de façon indépendante, sous la forme d'un dérivé ester, carbamate, carbonate ou sulfonate ;
R³ est un hydrogène, un alkyle, un amino, un monoalkylamino, un dialkylamino, un cycloalkyle, un aryle, un aralkyle, un halogénoalkyle, un hétéroalkyle, un cyanoalkyle, un alkylène-C(O)-R (où R est un hydrogène, un alkyle, un hydroxy, un alcoxy, un amino, un monoalkylamino ou un dialkylamino) ou un acyle ;
Ar¹ est un aryle ;
« aryle » désigne un reste hydrocarboné aromatique monovalent monocyclique ou bicyclique, optionnellement substitué, de façon indépendante, par un, deux ou trois substituants choisis dans le groupe constitué par un alkyle, un hydroxy, un alcoxy, un halogénoalkyle, un halogénoalcoxy, un hétéroalkyle, un halogéno, un nitro, un cyano, un amino, un monoalkylamino, un dialkylamino, un méthylènedioxy, un éthylènedioxy et un acyle ;
« acyle » désigne un reste -C(O)-R où R est un hydrogène, un alkyle, un cycloalkyle, un cycloalkylalkyle, un phényle ou un phénylalkyle ;
« hétéroalkyle » désigne un reste alkyle tel que défini ici où un, deux ou trois atomes d'hydrogène ont été remplacés par un substituant choisi de façon indépendante dans le groupe constitué par -OR^{a}, -NR^{b}R^{c} et -S(O)ₙR^{d} (ou n est un entier de 0 à 2), étant entendu que le point d'attachement du reste hétéroalkyle fait intervenir un atome de carbone, où R^{a} est un hydrogène, un acyle, un alkyle, un cycloalkyle ou un cycloalkylalkyle, R^{b} et R^{c} sont, de façon indépendante l'un de l'autre, un hydrogène, un acyle, un alkyle, un cycloalkyle ou un cycloalkylalkyle ou bien R^{b} et R^{c} forment ensemble un cycloalkyle ou un arylcycloalkyle ; et, si n est égal à 0, R^{d} est un hydrogène, un alkyle, un cycloalkyle ou un cycloalkylalkyle et, si n est égal à 1 ou 2, R^{d} est un alkyle, un cycloalkyle, un cycloalkylalkyle, un amino, un acylamino, un monoalkylamino ou un dialkylamino ;
« cycloalkyle » désigne un reste hydrocarboné cyclique saturé monovalent ayant 3 à 7 atomes de carbone cyclisés ; et
« alkyle » désigne un reste hydrocarboné linéaire saturé monovalent ayant 1 à 6 atomes de carbone ou un reste hydrocarboné ramifié saturé monovalent ayant 3 à 6 atomes de carbone,
ou sel pharmaceutiquement acceptable de celui-ci.

2. Composé selon la revendication 1, où Ar¹ est un phényle substitué de façon optionnelle.

3. Composé selon la revendication 2, où Ar¹ est un groupe phényle substitué de façon indépendante par un ou deux groupes halogéno, alkyle ou méthoxy.

4. Composé selon la revendication 3, où Ar' est le 2-chlorophényle, le 2-méthylphényle ou le 2-méthoxyphényle.

5. Composé selon la revendication 4, de formule :

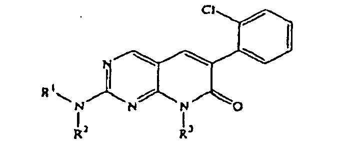

6. Composé selon l'une quelconque des revendications 2 à 5, où R¹ est un hydrogène ou un méthyle.

7. Composé selon la revendication 6, où R² est le (1,1-diméthyl-2-hydroxy)éthyle ou le (1,2-diméthyl-2-hydroxy)propyle, où chaque groupe hydroxy présent dans R² peut être, de façon indépendante, sous la forme d'un dérivé ester, carbamate, carbonate ou sulfonate.

8. Composition pharmaceutique comprenant un excipient pharmaceutiquement acceptable et un composé selon l'une quelconque des revendications 1 à 7.

9. Procédé pour la préparation d'un composé selon la revendication 1, comprenant les étapes de mise en contact d'un composé de formule Ig 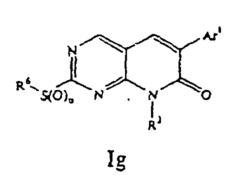 avec une amine de formule R¹R²NH dans des conditions suffisantes pour produire un composé de formule 1 : 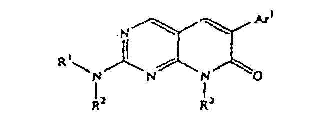 où
R¹, R², R³ et Ar¹ sont tels que définis dans la revendication 1 ;
n est un entier de 0 à 2 ; et
R⁶ est un groupe alkyle.

10. Procédé selon la revendication 9, où n est égal à 1.

11. Procédé selon la revendication 9, où n est égal à 2.

12. Procédé pour la production d'une pyrimidine de formule 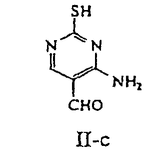 comprenant :
(a) la mise en contact d'un acétal de formule NC-CH₂-CH(OR^{a})₂, avec un formiate d'alkyle de formule HCO₂R en présence d'une base pour condensation dans des conditions suffisantes pour obtenir un produit condensé ; et
(b) la mise en contact dudit produit condensé avec de la thiourée en présence d'une base pour cyclisation dans des conditions suffisantes pour obtenir ladite pyrimidine de formule II-c,
où chacun des R et R^{a} est, de façon indépendante, un alkyle.

13. Procédé selon la revendication 12, dans lequel ladite base pour condensation est un tert-butylate.

14. Procédé selon la revendication 12, dans lequel ladite base pour cyclisation est un alcoolate.

15. Procédé pour la production d'une pyridopyrimidine de formule 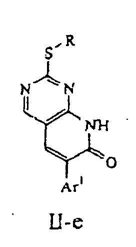 comprenant :
(a) la mise en contact d'une pyrimidine de formule 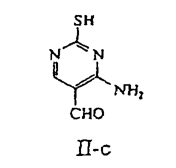 avec un agent alkylant de formule R-X¹ en présence d'une base alkylante dans des conditions suffisantes pour obtenir une pyrimidine alkylée de formule : 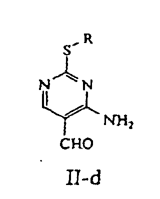 et
(b) la mise en contact de ladite pyrimidine alkylée de formule II-d avec un arylacétate de formule Ar¹-CH₂-CO₂R en présence d'une base pour cyclisation dans des conditions suffisantes pour obtenir ladite pyridopyrimidine de formule II-e ; ou
(a) la mise en contact de ladite pyrimidine de formule II-c avec ledit arylacétate d'aryle de formule Ar¹-CH₂-CO₂R en présence d'une base pour cyclisation dans des conditions suffisantes pour obtenir une pyridopyrimidine thiol de formule 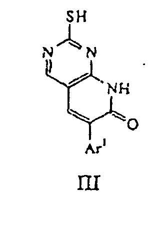
(b) la mise en contact dudit pyridopyrimidine thiol de formule III avec ledit agent alkylant de formule R-X¹ en présence d'une base alkylante dans des conditions suffisantes pour obtenir ladite pyridopyrimidine de formule II,
où
X¹ est un groupe partant ;
chaque R est, de façon indépendante, un alkyle ;
Ar¹ est un aryle.

16. Procédé selon la revendication 15, dans lequel X¹ est un halogénure.

17. Procédé selon la revendication 15, comprenant en outre la mise en contact de ladite pyridopyrimidine de formule II avec un agent alkylant de l'azote de formule R³-X² dans des conditions suffisantes pour obtenir une pyridopyrimidine N-substituée de formule 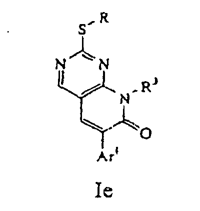 où
R³ est un alkyle, un amino, un monoalkylamino, un dialkylamino, un cycloalkyle, un aralkyle, un halogénoalkyle, un hétéroalkyle, un cyanoalkyle, un alkylène-C(O)-R' (où R' est un hydrogène, un alkyle, un hydroxy, un alcoxy, un amino, un monoalkylamino ou un dialkylamino) ou un acyle ;
X² est un groupe partant ; et
Ar¹ et R sont tels que définis dans la revendication 15.

18. Procédé selon la revendication 17, dans lequel X² est un halogénure.

19. Composés selon l'une quelconque des revendications 1 à 7, en tant qu'agents thérapeutiquement actifs.

20. Utilisation d'un ou plusieurs composés selon l'une quelconque des revendications 1 à 7, pour la préparation d'un médicament pour la prévention ou le traitement d'une arthropathie, de la maladie de Crohn, de la maladie d'Alzheimer, du syndrome du colon irritable, du syndrome de détresse respiratoire de l'adulte ou de la bronchopneumopathie chronique obstructive.

21. Composé selon la revendication 1, ayant la formule 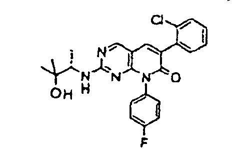 et ses sels pharmaceutiquement acceptables.

22. Composé selon la revendication 1, ayant la fonnule 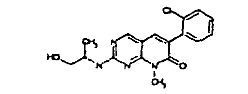 et ses sels pharmaceutiquement acceptables.

23. Composé selon la revendication 1, ayant la formule 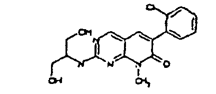 et ses sels pharmaceutiquement acceptables.
